(19) Europäisches Patentamt
European Patent Office
Office européen des brevets

(11) **EP 4 413 920 A1**

(12) **EUROPEAN PATENT APPLICATION**

(43) Date of publication:
**14.08.2024  Bulletin 2024/33**

(21) Application number: **23155692.9**

(22) Date of filing: **08.02.2023**

(51) International Patent Classification (IPC):
**A61B 5/0295** (2006.01)    **A61B 5/026** (2006.01)
**A61B 5/024** (2006.01)    **A61B 5/1455** (2006.01)
**A61B 5/00** (2006.01)

(52) Cooperative Patent Classification (CPC):
**A61B 5/0295; A61B 5/02416; A61B 5/0261;
A61B 5/14551; A61B 5/6801;** A61B 2562/046

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Vrije Universiteit Brussel
1050 Brussel (BE)**
• **Universidad de Oriente
90500 Santiago de Cuba (CU)**

(72) Inventors:
• **MORILLO, Ángel Solé
28016 Madrid (ES)**
• **STIENS, Johan
2820 Bonheiden (BE)**
• **GOMES, Bruno Tiago da Silva
1180 Brussels (BE)**
• **CAUSE, Joan Lambert
90100 Santiago de Cuba (CU)**
• **NARANJO, Juan Carlos García
90300 Santiago de Cuba (CU)**

(74) Representative: **Calysta NV
Lambroekstraat 5a
1831 Diegem (BE)**

(54) **IMPROVED PPG MEASUREMENT**

(57)    Device for measuring a photoplethysmogram (PPG) of a tissue, the device comprising:
a tissue contact surface (2) configured to be contacted by the tissue;
a plurality of light emitter units (6) for emitting light in the tissue contact surface (2), wherein the plurality of light emitter units (6) comprise at least three light emitter units (6);
a plurality of photodetectors (7) for detecting in the tissue contact surface (2) the light emitted by the light emitter units (6), wherein the plurality of photodetectors (7) comprise an at least three photodetectors (7);
a signal unit for measuring PPG signals of different optical paths provided by all possible pairs of light emitter units (6) and photodetectors (7).

Fig. 2A

## Description

### Technical Field

The present invention relates to the measurement of a photoplethysmogram (PPG).

### Prior art

The PPG is the optically obtained plethysmogram. The plethysmogram is the measurement signal representing the changes in volume within an organ or a body (usually resulting from fluctuations in the amount of blood or air it contains). The PPG is particularly used to detect blood volume changes in the microvascular bed of tissue. The PPG waveform comprises a pulsatile (often called 'AC') physiological waveform which is superimposed on a much larger much larger slowly varying quasi-static ('DC') baseline. The PPG allows theoretically to determine many physiological parameters or vital signals like the heart rate, pulse rate variability, blood pressure, respiration, oxygen level of the blood, etc. However, the PPG is often not very robust and depends on many factors like probe contact force, skin and ambient temperature, skin tone, sensor position in the body, motion artefacts, sensor contact with skin, etc. Different solutions have been proposed to the individual problems.

EP3342336A1 discloses a PPG sensor with an additional touch sensor for detecting the presence and contact area of the tissue on the sensor and with an additional force sensor to detect the contact force of the tissue. The touch sensor is preferably a capacitive sensor. The contact area and the contact force allow to determine the contact pressure. It is proposed to control the contact pressure to provide the optimal signal. In one embodiment, a location sensitive capacitive sensor array (similar to a touch screen) is overlaying a matrix of PPG sensors. The capacitive sensor array allows to detect a contact pressure profile and select the best located PPG sensor to measure the PPG based on the contract pressure profile. However, such location sensitive pressure sensors are complex.

WO2018231672A1 discloses the arrangement of a plurality of PPG sensors arranged radially around a central photodetector. Each PPG sensor has a plurality of photoemitters and one peripheral photodetector. Each PPG sensor forms a channel pair by the peripheral photodetector and one of the emitters of the respective PPG sensor. Each PPG sensor comprises a short path and a long path. The function of the central detector is not disclosed. The best PPG sensor for the PPG measurement is selected based on the SNR of the channel pairs of the different PPG sensors. This allows to select best location of the PPG measurement based on the SNR.

Nevertheless, it has not yet been found simple PPG device which robustly determines the PPG of tissue for different types of tissue and/or for different types of ambient physical conditions.

### Brief summary of the invention

It is the object of the invention to improve the PPG measurements of the state of the art and/or to provide a PPG device working for many different kinds of people.

The inventors found many improvements for PPG devices which are described below. Those improvements are particularly advantageous in combination but can also be used individually to improve PPG devices.

An improvement (Improvement 1) is a device for measuring a PPG of a tissue, the device comprising: a tissue contact surface configured to be contacted by the tissue; a plurality of light emitter units for emitting light in the tissue contact surface, wherein the plurality of light emitter units comprise at least three light emitter units; a plurality of photodetectors for detecting in the tissue contact surface the light emitted by the light emitter units, wherein the plurality of photodetectors comprise at least three photodetectors; a signal unit for measuring PPG signals of different optical paths provided by all pairs of light emitter units and photodetectors, a processor configured to compute a (first) processed parameter based on at least one of the PPG signals.

By using a plurality (at least 3) of light emitter units and a plurality of photodetectors (at least 3) and by configuring the signal unit to allow to measure independently each optical path provided by all possible pairs of the light emitter units and photodetectors, the available components are optimally used to measure a multitude of optical paths at many different distances and locations on the contact surface. This allows to find the one or more optical paths for one or more optimal PPG signals for each type of tissue for a derivation of one or more particular physiological signals or vital parameters

To clarify what all possible pairs means: If one photodetector or light emitter unit is not used in combination with all light emitter units and photodetectors, respectively, of the device, such a photodetector or light emitter unit should not be considered as part of the plurality of photodetectors of the claim or of the plurality of light emitter units of the claim, respectively. Thus, a device with ten photodetectors and ten light emitter units, where 9 photodetectors are used in combination with all ten photodetectors and one photodetector is used just in combination with one light emitter unit or with none would still fall under the scope of protection of the above claim.

A further improvement (Improvement 2) is a device for measuring a PPG of a tissue, the device comprising: a tissue contact surface configured to be contacted by the tissue; a plurality of light emitter units for emitting light in the tissue contact surface; a plurality of photodetectors for detecting in the tissue contact surface the light emitted by the light emitter units; a signal unit for measuring PPG signals of different optical paths provided by different pairs of light emitter units and photodetectors, wherein the plurality of light emitter units and the plurality of photodetectors are arranged in a grid of rows and columns.

The arrangement in a grid has the big advantage that the emitter units and the photodetectors are well distributed over the tissue contact surface, can be well controlled and allow to realize nearly any distance between the different pairs of emitter unit and photodetector. This allows thus a PPG sensor which is configured to measure nearly all parameters you can compute from PPG signals and can be used in relation for nearly any type of tissue.

A further improvement (Improvement 3) is a device for measuring a PPG of a tissue, the device comprising: a tissue contact surface configured to be contacted by the tissue; a PPG sensor arranged in the tissue contact surface and configured to measure a PPG signal of the tissue; an auxiliary sensor configured to measure at least one measurement parameter influencing the PPG signal; a processing means configured to perform a signal processing, wherein the signal processing comprises to compute periodically a (first) processed parameter based on the PPG signal continuously measured, wherein the correctness of the processed parameter is verified based on the auxiliary parameter measured with the auxiliary sensor.

This significantly improves the reliability of the PPG device. PPG devices normally trigger an alarm for certain changes in the PPG signal caused by physiological changes. Thus, the auxiliary sensor helps to detect, if the change in the PPG signal is due to a physiological change or due to a change in other conditions measured by the auxiliary sensor. Thus, the change in the PPG signal causes only an alarm, if the change is not caused by a change in the measurement parameter measured by the auxiliary sensor.

A further improvement (Improvement 4) is a device for measuring a photoplethysmogram (PPG) of a tissue, the device comprising: a tissue contact surface configured to be contacted by the tissue; a PPG sensor arranged in the tissue contact surface and configured to measure a PPG signal of the tissue; a capacitive sensor arranged in the tissue contact surface and configured to measure a capacitance of the tissue.

The combination of the PPG sensor and the capacitive sensor is highly advantageous. The capacitive sensor can be used for a multitude of functions which cannot be realized by the PPG sensor (or not with sufficient accuracy) or can be realized with a much lower power consumption like contact detection, distance detection, respiration signal detection, tonometric signal detection and many more. These functions can be used to filter the PPG signal, to increase their precision, to calibrate the device, etc.

A further improvement (Improvement 5) is a device for measuring a PPG of a tissue, the device comprising: a tissue contact surface configured to be contacted by the tissue; a PPG sensor arranged in the tissue contact surface; wherein a skin tone of a skin of the tissue is measured based on the PPG signal obtained by the PPG sensor.

The measurement of the skin tone of the skin of the tissue with the PPG sensor allows to correct the PPG signals by the skin tone or to calibrate the device based on the measured skin tone.

A further improvement (Improvement 6) is a device for measuring a PPG of a tissue, the device comprising: a tissue contact surface configured to be contacted by the tissue; a PPG sensor arranged in the tissue contact surface and configured to measure PPG signals of a plurality of optical paths; and a processing means configured to perform a signal processing to compute a (first) processed parameter, wherein the processing means is configured to perform a channel calibration, wherein the channel calibration comprises to : measure a PPG signal of all optical paths of a full set of optical paths of the (first) processed parameter, calculate a quality (index) indicator for all optical paths based on their PPG signals, and select a sub-set of optical paths based on the quality (index) indicators of the optical paths; wherein the signal processing comprises to: measure the PPG signal(s) of the sub-set of optical paths, and derive one or more processed parameters based on the PPG signal(s) of the sub-set of optical paths measured.

The automatic selection of optical paths based on a quality (index) indicator of the PPG signals allows to automatically detect the best optical path(s) for a measurement of one or more physiological or vital parameters (processed parameters).

A further improvement (Improvement 7) is a device for measuring a PPG of a tissue, the device comprising: a tissue contact surface configured to be contacted by the tissue; a PPG sensor arranged in the tissue contact surface and configured to measure a PPG signal of the tissue; and a processing means configured to perform a signal processing to compute a (first) processed parameter based on the PPG signal, wherein the processing means is configured to perform a mechanical tissue calibration, wherein the mechanical tissue calibration comprises to measure at least one PPG calibration signal with the PPG sensor and optionally an auxiliary calibration signal with an auxiliary sensor while the pressure of the tissue applied on the tissue contact surface is changed, wherein the mechanical tissue calibration comprises to detect different mechanical zones of the tissue based on the PPG calibration signal or based on the auxiliary calibration signal, wherein the mechanical tissue calibration comprises further storing at least one signal quality index indicator derived from the waveform of the PPG calibration signal for each detected mechanical zone and/or at least one waveform of the PPG calibration signal for each detected mechanical zone, wherein the signal processing comprises the measurement of a PPG measurement signal by the PPG sensor and a detection of the mechanical zone of the tissue during PPG measurement signal based on the mechanical tissue calibration, preferably based on the stored signal quality index indicators and/or waveforms of the different mechanical zones.

While the state of the art did either for each measurement the scan of the full applied pressure range to find the optimal application pressure or correct the pressure based on some non-tissue specific models, which either led to very long measurement procedures or unprecise measurements. This improvement proposes a mechanical tissue calibration for having a quick and precise PPG measurement afterwards independent of the applied pressure, but nevertheless adapted to the individual tissue. Instead of detecting the applied pressure by some pressure measurements to detect the mechanical zone of the tissue, which is not precise due to the hysteresis effect, the current improvement proposes to use the waveform to detect the mechanical zones. Once the mechanical zone is detected, the PPG measurement could be corrected and/or the applied pressure could be changed.

A further improvement is a device for measuring a PPG of a tissue, the device comprising : a tissue contact surface configured to be contacted by the tissue; a PPG sensor arranged in the tissue contact surface, wherein the device is characterized by one or more of the following embodiments and/or one or more of the previous improvements.

A further improvement is a method for measuring a photoplethysmogram (PPG) of a tissue with a PPG sensor, the method comprising: placing a tissue on a tissue contact surface; and measuring at least one PPG signal of the tissue with the PPG sensor, wherein the device is characterized by the corresponding method features of one or more of the following embodiments and/or one or more of the previous improvements.

A further improvement is a computer program comprising instruction configured to execute the following steps, when executed on a processing means: controlling a PPG device with a PPG sensor characterized by the features of one or more of the following embodiments and/or one or more of the previous improvements.

A further improvement is a computer program comprising instructions configured to execute the following steps, when executed on a processing means: receiving a PPG signal; and computing a (first) processed parameter based on the received PPG signal, characterized by the features of one or more of the following embodiments and/or one or more of the previous improvements.

The following embodiments show further features of the previously disclosed improvements.

In one embodiment, the PPG sensor comprises : a light emitter unit (preferably a plurality thereof) for emitting light in the tissue contact surface ; a photodetector (preferably a plurality thereof) for detecting in the tissue contact surface the light emitted by the light emitter; a signal unit for outputting /measuring a PPG signal of the tissue based on the light detected at the photodetector

In one embodiment, the device comprising a plurality of light emitter units for emitting light in the tissue contact surface, wherein the light emitter unit is a first light emitter unit of the plurality of light emitter units.

In one embodiment, the device comprising a plurality of photodetectors for detecting in the tissue contact surface light emitted by the light emitter unit, wherein the photodetector is a first photodetector of the plurality of photodetectors.

In one embodiment, the plurality of photodetectors is located at different locations in the tissue contact surface so that the photodetectors at different locations provide different optical paths with respect to the light emitter unit.

In one embodiment, the device comprising a plurality of light emitter units for emitting light in the tissue contact surface, wherein the light emitter unit is a first light emitter unit of the plurality of light emitter units, wherein the light emitter units are located at different locations, wherein each photodetector is configured to measure independently the light from each light emitter unit, and the signal unit is configured to process a PPG signal of each possible optical path between the plurality of light emitting units and the plurality of photodetectors.

In one embodiment, the plurality of light emitter units and the plurality of photodetectors are arranged in a grid of rows and columns.

In one embodiment, a row and/or a column of the grid comprises alternately a light emitter unit and a photodetector. Preferably, a neighbouring row and/or column of the row and/or column show the same pattern off-set by one position

In one embodiment, the device can measure (or select among) a plurality of different optical paths, wherein the plurality of different optical paths comprises at least one optical path between each emitter-detector couple of the plurality of light emitter units and the plurality of photodetectors, wherein the plurality of photodetectors comprises at least three photodetectors.

In one embodiment, the device can measure (or select among) a plurality of different optical paths, wherein the plurality of photodetectors comprises further a second photodetector and a third photodetector, wherein the plurality of different optical paths comprise a first optical path from the first light emitter unit to the first photodetector, a second optical path from the first light emitter unit to the second photodetector and a third optical path from the first light emitter unit to the third photodetector.

In one embodiment, each of the light emitter units is configured to emit the light of the same spectral band.

In one embodiment, wherein each of the plurality of light emitter units comprise a spectral number of sub-light emitter units emitting light of a different spectral band, wherein the plurality of different optical paths comprises the spectral number of optical paths corresponding to the different sub-light emitter units between each emitter-detector couple of the plurality of light emitter units and the plurality of photodetectors.

In one embodiment, the light emitter unit (or each of the plurality of light emitter units or each of the sub-light emitter units) are configured to emit light at a first light intensity and at a second light intensity different from the first light intensity, wherein the plurality of different optical paths comprises for each emitter-detector couple at least one first intensity optical path using emitted light at the first light intensity and at least one second intensity optical path using emitted light at the second intensity. Different light intensities are particular advantageous, as some tissue types absorb more light than others. So, the light intensity adaption can help to obtain high quality PPG signals. This is particularly advantageous in combination with the plurality of optical paths at different distances. So, the longer distances might be measured at a higher quality with a higher light intensity than shorter distances.

In one embodiment, the processing means is configured to : measure a PPG signal of all optical paths of a full set of optical paths, calculating a quality index indicator for all optical paths based on their PGG signals, selecting a sub-set of optical paths based on the quality indicators of the optical paths, measure the PPG signal(s) of the sub-set of optical paths. The full set of optical paths include preferably light emitter unit - photodetector- pairs of different distance, different wavelengths and different light intensities emitted by the light emitter units.

In one embodiment, the device or the processing means is configured to compute a plurality of different processed parameters, wherein a different sub-set of optical channels is selected for computing the different processed parameters.

In one embodiment, the device comprises a plurality of row detector conductors, a plurality of column detector conductors, a detector switching means and signal acquisition electronics, wherein the plurality of photodetectors comprises a plurality of column sub-groups of photodetectors with each column sub-group connected by a different column detector conductor, wherein the plurality of photodetectors comprises a plurality of row sub-groups of photodetectors with each row sub-group connected by a different row detector conductor, wherein the detector switching means is configured to switch one of the row detector conductors and one of the column detector conductors to the signal acquisition electronics to detect the signal measured at the intersecting photodetector being in the row sub-group connected by the row detector conductor through the detector switching means to the signal acquisition electronics and being in the column sub-group connected by the column detector conductor through the detector switching means to the signal acquisition electronics.

In one embodiment, the device comprises a plurality of row emitter conductors, a plurality of column emitter conductors, an emitter switching means and driver electronics, wherein the plurality of light emitting units comprises a plurality of column sub-groups of light emitting units with each column sub-group connected by a different column emitter conductor, wherein the plurality of light emitting units comprises a plurality of rowsub-groups of light emitting units with each rowsub-group connected by a different row emitter conductor, wherein the emitter switching means is configured to switch one of the row emitter conductors and one of the column detector conductors to the driver electronics to provide a drive current to the intersecting light emitting unit being in the row sub-group connected by the row emitter conductor through the emitter switching means to the driver electronics and being in the column sub-group connected by the column emitter conductor through the emitter switching means to the driver electronics.

This arrangement of the control of the photodetectors and/or light emitter units in a grid control allows to use the same acquisition and/or driver circuits for the plurality of photodetectors and/or light emitter units. The organization of the electrodes does not require that the photodetectors are actually arranged in rows and columns, just that the control of the photodetectors is like in a grid.

In one embodiment, the device comprises a capacitive sensor.

In one embodiment, wherein the capacitive sensor comprises at least one capacitive electrode arranged in the tissue contact surface, wherein the capacitive sensor is configured for each capacitive electrode to apply a drive signal generated by a drive circuit on the capacitive electrode and measure the capacitive value of the capacitive electrode on the same capacitive electrode by an acquisition circuit such that the drive circuit and the acquisition circuit are galvanically connected by the capacitive electrode.

In one embodiment, a parameter is measured based on the capacitive sensor, wherein the parameter comprises one of the following : presence of the tissue in the vicinity of the tissue contact surface ; detection of a contact of the tissue with the tissue contact surface ; area of contact of the tissue on the tissue contact surface; the respiration signal; the tonometric signal; an elastic tissue behavior, and a contact pressure of the tissue on tissue contact surface. These parameters are preferably determined by the processing means.

In one embodiment, the parameter is measured based on a combination of the capacitive sensor and one or more of the PPG sensor and a contact force sensor.

In one embodiment, the device comprises a contact force sensor (being different than the capacitive sensor) for measuring the contact force of the tissue on the tissue contact surface.

In one embodiment, the device comprises a temperature sensor in the tissue contact surface to detect the temperature of the tissue.

In one embodiment, the device comprises an inertial sensor in device to detect the movement of the tissue.

In one embodiment, the device comprises a processing means.

In one embodiment, the processing means is configured to process the PPG signal(s) to obtain at least one processed parameter.

In one embodiment, the processing means is configured to process the PPG signal(s) and the signals of the auxiliary sensor(s) to obtain at least one processed parameter.

In one embodiment, a skin tone of the tissue is measured based on the PPG signal obtained by the PPG sensor.

In one embodiment, the skin tone is measured with the following steps : positioning the tissue at a distance to the tissue contact surface or in contact on the tissue contact surface; emitting from the light emitting unit light of a first spectral band and measuring the emitted light reflected from the tissue at the photodetector as first reflected tone light; emitting from the light emitting unit or another light emitting unit of the plurality of light emitting units light of a second spectral band and measuring the emitted light reflected from the tissue at the photodetector or at another photodetector of the plurality of photodetectors as second reflected tone light; computing the skin tone based on the first and second reflected tone light. This is a very simple way to measure the skin tone of the tissue.

In one embodiment, the tissue is positioned, during the measurement of the first and second reflected tone light, in contact with the tissue contact surface. Preferably, the first reflected tone light can be obtained from the DC component of the PPG signal of the first spectral band and the second reflected tone light can be obtained from the DC component of the PPG signal of the second spectral band. This is less precise, but does not require the user to exercise an extra positioning of the tissue only for the measurement of the skin tone.

In one embodiment, the tissue is positioned, during the measurement of the first and second reflected tone light, at a distance to the tissue contact surface. This allows a more precise measurement, but is more inconvenient for the user.

In one embodiment, the positioning of the tissue in the correct distance is supported by a distance measurement realized based on the capacitive sensor. This helps to measure the skin tone with a higher precision.

In one embodiment, the skin tone is measured with the further steps : emitting from the light emitting unit light of a third spectral band and measuring the emitted light reflected from the tissue at one of the photodetector(s) as third reflected tone light; computing the skin tone based on the first, second and third reflected tone light. In one embodiment, the measured skin tone is used to calibrate the device for the PPG measurement of the tissue. This allows to provide PPG signals with high quality for each type of skin tone.

In one embodiment, the mechanical zones comprise an optimal mechanical zone and further mechanical zones, wherein the mechanical calibration comprises to determine a correction function for each further mechanical zone based on the waveform of the respective further mechanical zone and the waveform of the optimal mechanical zone, wherein the correction function of the further mechanical zone defines how the waveform of the further mechanical zone must be changed to obtain the waveform of the optimal mechanical zone, wherein the signal processing comprises to apply the correction function of the determined mechanical zone on the PPG measurement signal, preferably on the waveform(s) of the PPG measurement signal.

In one embodiment, the device is configured, based on the detected mechanical zone, to change the applied pressure of the tissue on the tissue contact surface so that the mechanical properties of the tissue will be in the optimal mechanical zone. The applied pressure can be changed actively a variable pressure means or could be (passively) changed by outputting user instructions how to change the applied pressure to get in the optimal mechanical zone.

In one embodiment, the PPG sensor comprises a plurality of photodetectors and a plurality of emitter units, wherein the full set of optical paths include light emitter unit- photodetector- pairs of different distance and/or different light intensities emitted by the light emitter units. The automatic selection of optical paths of different lengths and/or different light intensities allows to find dynamically the best optical path(s) for each measurement.

In one embodiment, the signal processing comprises to compute a second processed parameter, wherein the channel calibration comprises to: measure a PPG signal of all optical paths of a full set of optical paths for the second processed parameter (second full set), calculate a quality (index) indicator for all optical paths based on their PPG signals, and select a second sub-set of optical paths based on the quality (index) indicators of the optical paths; wherein the signal processing comprises to: measure the PPG signal(s) of the second sub-set of optical paths, and determine the second processed parameter based on the PPG signals of the second sub-set of optical paths measured. The second full set refers to a set of optical paths pre-selected to be potentially relevant for the measurement of the second processed parameter. The full set of optical paths for the (first) processed parameter (first full set) refers a set of optical paths pre-selected to be potentially relevant for the measurement of the (first) processed parameter. Preferably, the overlapping optical paths of the full sets of optical paths for the (first) processed parameter and the second processed parameter are measured only once. Preferably, the overlapping optical paths of the sub-set of optical paths and the second sub-set of optical paths are measured in the signal processing only once and can be used for both the processed parameter and the second processed parameter. The dynamical selection of optical paths is particularly advantageous, when the PPG device can compute different processed parameters. In this case, for each processed parameter, a different sub-set of optical paths can be determined based on the quality indicators of the optical channel.

In one embodiment, to verify the processed parameter, the processing means determines a change in the PPG signal and verifies based on the auxiliary parameter measured with the auxiliary sensor, if the change in the PPG signal is caused by a change in the measurement parameter or based on a physiological change of the tissue.

In one embodiment, the auxiliary sensor comprises one or more of a capacitive sensor, a contact force sensor, a temperature sensor and an inertial sensor. Especially, when those auxiliary sensors are combined, the PPG device gets more independent against more measurement conditions influencing the PPG signal so that the result of the processed parameter is more reliable.

In one embodiment, the PPG sensor comprises a plurality of optical paths,

In one embodiment, the capacitive sensor is arranged in the tissue contact surface and configured to measure a capacitance of the tissue.

In one embodiment, a contact force sensor is configured to detect a contact force of the tissue against the tissue contact surface.

In one embodiment, a temperature sensor is configured to measure the external temperature and/or the temperature of the tissue.

In one embodiment, an inertial sensor is configured to detect a movement of the device.

In one embodiment, the improvement 3 and improvement 6 described above are combined, wherein, if the verification of the processed parameter based on the auxiliary measurement results that the processed parameter is not trustworthy, the processing device is configured to re-perform the channel calibration to select a new sub-set of optical channels for the processed parameter, wherein after the re-performed channel calibration, the processing device is configured to continue with the signal processing using at least one PPG signal of the new sub-set of optical paths. The re-calibration of the channels used for the processed parameter based on the auxiliary measurement of an auxiliary sensor allows to obtain even for longer measurements a reliable processed parameter.

In one embodiment, the PPG device is a wearable device which continuously monitors the processed parameter. Preferably the continuous monitoring lasts longer than 1 minute, preferably longer than 5 minutes, preferably longer than 10 minutes, preferably longer than an hour.

Other embodiments according to the present invention are mentioned in the appended claims and the subsequent description of an embodiment of the invention.

## Brief description of the Drawings

Fig. 1 shows a schematic diagram of an embodiment for a device 1 for measuring a PPG.

Fig. 2A and B shows two sides of a substrate of an exemplary realization of the PPG device with the PPG sensor, the capacitive sensor and the contact force sensor.

Fig. 3 shows a cross-sectional view along line A of the realization of Fig. 2A and B.

Fig. 4 shows a detailed view of the exemplary light emitter units used in the present embodiments.

Fig. 5A and B shows two sides of a substrate of a further exemplary realization of the PPG device with the PPG sensor, the capacitive sensor and the contact force sensor.

Fig. 6 shows a cross-sectional view of another embodiment of the PPG device.

Fig. 7 shows an exemplary circuit diagram of the plurality of photodetectors in the PPG sensor.

Fig. 8 shows an exemplary circuit diagram of a common signal acquisition for the plurality of photodetectors in the PPG sensor.

Fig. 9 shows an exemplary circuit diagram of the plurality of light emitting units in the PPG sensor.

Fig. 10 shows the principle of operation of optical channels.

Fig. 11 shows a representation of the penetration depth of the optical channels as a function of the wavelength..

Fig. 12 shows the principle of the capacitive measurement by the capacitive sensor.

Fig. 13 A-C show different configurations of the capacitive electrode of the capacitive sensor.

Fig. 14 shows the capacitive sensor including a protective shielding area.

Fig. 15 illustrates the principle used for measuring the contact area and the contact pressure.

Fig. 16 illustrates the principle used to measure the tonometric signal.

Fig. 17 shows the elastic and viscoelastic behaviour of the tissue in the capacitive signal obtained by the capacitive sensor.

Fig. 18 shows the steps for an exemplary method of a skin tone measurement.

Fig. 19 shows the steps for an exemplary method of a channel calibration.

Fig. 20 shows the steps for an exemplary method of a mechanical tissue calibration.

Fig. 21 shows the pressure scale and the optimal operation zone.

Fig. 22 and 23 shows the PPG signals during the mechanical tissue calibration.

Fig. 24 shows the steps for an exemplary method of a operation mode of the PPG device.

In the drawings, the same reference numbers have been allocated to the same or analogue element.

## Detailed description of an embodiment of the invention

Other characteristics and advantages of the present invention will be derived from the following non-limitative description, and by making reference to the drawings and the examples.

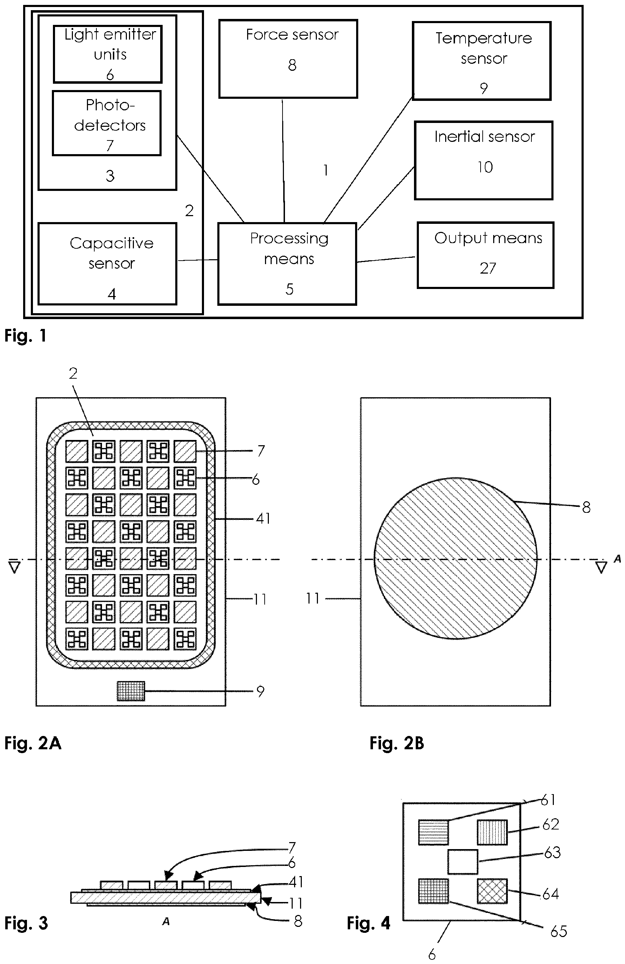

Fig. 1 shows an embodiment for a device 1 for measuring a PPG. The device 1 comprises a tissue contact surface 2 and a PPG sensor 3 and a processing means 5. In a preferred embodiment, the device 1 comprises further at least one auxiliary sensor. The at least one auxiliary sensor can comprise one or more of a capacitive sensor 4, a force sensor 8, a temperature sensor 9 and an inertial sensor 10.

The tissue contact surface 2 is configured to receive a tissue of a body or user for performing the PPG measurement of the tissue. The tissue can be placed on the tissue contact surface 2 or vice versa the tissue contact surface 2 can be placed on the tissue. The tissue contact surface 2 is preferably a flat surface. However, the tissue contact surface 2 can also comprise a curved or otherwise non-flat surface. Such a non-flat surface can have the negative shape of the body portion where the PPG shall be measured. The body portion can be a palm of a hand, a finger or toe, etc. But preferably, the tissue contact surface 2 is generally applicable for several body locations. This is why a flat tissue contact surface 2 is preferred. In the shown embodiment, the tissue contact surface 2 is made of a rigid material. However, the tissue contact surface 2 can also be made of a flexible, semi-rigid or adaptable material so that the tissue contact surface 2 can adapt to the shape of the tissue. The tissue contact surface is preferably configured for a reflective PPG measurement. The tissue contact surface is preferably one connected surface. However, the tissue contact surface could also comprise two tissue contact sub-surfaces, e.g. for realizing a transmissive PPG measurement. In the latter case, the two tissue contact sub-surface are arranged on two opposed sides of a tissue, e.g. a finger or toe. The tissue refers preferably to a human body. However, it is also possible that the tissue refers to an animal body.

The PPG sensor 3 is configured to measure a PPG signal of a tissue placed on the tissue contact surface 2. The PPG sensor 3 is arranged in the tissue contact surface 2. The PPG sensor 3 comprises a light emitter unit 6 and a photodetector 7. In a preferred embodiment, the PPG sensor 3 comprises a plurality of light emitter units 6 and a plurality of photodetectors 7 as shown for example in the embodiment of Fig. 2A.

The light emitting unit 6 is configured to emit light. The light is configured to be emitted in the tissue contact surface 2 so that, when tissue is placed on the tissue contact surface 2, the light emitting unit 6 can emit light in the tissue. In some embodiment, the light emitting unit 6 is also configured for certain measurements to illuminate the tissue surface, when the tissue is in the vicinity of (but not in contact with) the tissue contact surface 2. The wavelength of the light emitting unit 6 is preferably above 100 nm, preferably above 200 nm, preferably above 300 nm, preferably above 400nm. The wavelength of the light emitting unit 6 is preferably below 1500 nm, preferably below 1400 nm, preferably below 1300 nm, preferably below 1200nm, preferably below 1 100nm. Thus, the emitted light is preferably visible and/or infrared light, the latter preferably near infrared light. However, the invention is not limited to this spectral range. The light emitting unit 6 is preferably configured to emit light in a specific spectral bandwidth. The spectral bandwidth is preferably smaller than 200 nm, preferably smaller than 150 nm, preferably smaller than 100 nm. The spectral bandwidth is defined as the band width of light at one-half the peak maximum. The light emitting unit 6 comprises at least one light emitter 61 to emit the light. The light emitter(s) 61, 62, 63, 64, 65 are preferably light emitting diodes (LEDs). However, other light emitting technologies like low-energy lasers can also be used. Light emitters with an angle of visibility of at least 45° are preferably used.

Preferably, the light emitting unit 6 is preferably configured to emit light with different spectral characteristics. Preferably, the light emitting unit 6 comprises a plurality of different light emitters (sub-units) 61, 62, 63, 64, 65 emitting each light with a different spectral characteristic and/or at a different peak wavelength. Fig. 4 shows an example with five light emitters 61, 62, 63, 64, 65 with different peak wavelengths. However, the light emitting unit 6 can also have two, three, four, six or more light emitters 61, 62, 63, 64, 65 at different peak wavelengths. The light emitter(s) is/are selected preferably such that they have a peak wavelength of one of the following spectral ranges: 800 nm to 1100 nm (infrared), 650-800 nm (red), 600-650 nm (yellow to red), 550-600 nm (green to yellow), 500-550 nm (green), 450-500 nm (blue to green) and 400-450 nm (violet to blue). Different light emitters 61, 62, 63, 64, 65 are preferably chosen such that each light emitter has a peak wavelength in a different spectral range. The different wavelengths are used to reach several vascular depths, enabling the monitoring particular tissue characteristics. For example, it is possible to study the physiological and/or pathological characteristics of arterioles, veins and arteries in a differentiated way. The number of wavelengths active during a predetermined period of time will be determined by the physiological parameter under evaluation, as well as by the particular device operating mode. The light emitters 61, 62, 63, 64, 65 could be combined into a single multiwavelength package or arranged in groups of individual light emitters. The different light emitters 61, 62, 63, 64, 65 described and their respective functions can also be realized by one light emitter which is able to emit the different spectral ranges described, e.g. by a variable light emitter emitting the different spectral ranges described. Such a variable light emitter could also be realized by a broad spectral light emitter which is filtered by a variable filter for obtaining the different spectral ranges. Even when describing subsequently and in the claims, different light emitters of the light unit 6, it shall always comprise as well, this option to realize these different light emitters with a single or more (but less than the light emitters) light source(s) with a variable light emission.

The description of the light emitting unit 6 above applies to all the plurality of light emitting units 6. The different light emitting units 6 are placed at different locations on the tissue contact surface 2 so that the PPG could be measured at different locations at the tissue. The different light emitting units 6 (at the different locations) are preferably (all) identical to each other. However, it is also possible that they distinguish (further to its locations) among each other.

The plurality of light emitting units 6 comprise preferably at least two light emitting units 6 with a first light emitting unit 6 and a second light emitting unit 6. The plurality of light emitting units 6 comprise preferably at least three light emitting units 6 with a third light emitting unit 6. The plurality of light emitting units 6 comprise preferably at least four light emitting units 6 with a fourth light emitting unit 6. The plurality of light emitting units 6 comprise preferably at least five light emitting units 6 with a fifth light emitting unit 6.

The photodetector(s) 7 is/are configured to detect light with the spectral characteristics of the light emitted by the light emitting unit 6, preferably by all light emitters 61, 62, 63, 64, 65. The photodetector(s) 7 is/are configured to detect light emitted by the light emitting unit 6 after having passed the tissue in contact with the tissue contact surface 2. In a preferred embodiment, the photodetector(s) 7 is/are configured to detect light emitted by the light emitting unit 6 and reflected/scattered back from the tissue to the photodetector(s) 7 (reflective PPG measurement). In a less preferred embodiment, the photodetector(s) 7 is/are configured to detect light emitted by the light emitting unit 6 and transmitted through the tissue to the photodetector(s) 7 (transmissive PPG measurement). For some measurements, the photodetector(s) 7 is/are configured to detect light emitted by the light emitting unit 6 and reflected from the surface of the tissue (arranged at a small distance to the tissue contact surface) to the photodetector(s) 7.

The photodetectors 7 detect the light photons from the tissue and produce an electrical signal proportional to the number of photons detected over time. Several types of photodetectors, such as photocells, photoresistors, photodiodes, and phototransistors, are adequate to be used with the device 1. The photodetectors 7 can be analog, digital or mixed type. Silicon photodiodes with wide spectral sensitivity range to accommodate the peak wavelengths of the used light emitters are preferred. It is also possible to employ photodetectors with relative spectral sensitivity that cover only a specific wavelength or a narrow set of wavelengths. In such a way that a set of these photodetectors can be grouped to form detection units associated with a set of light emitters. A miniature planar housing is the preferred one, as it provides smaller dark currents and lowers current noise.

The plurality of photodetectors 7 comprise preferably at least two photodetectors 7 with a first photodetector 7 and a second photodetector 7. The plurality of photodetectors 7 comprise preferably at least three photodetectors 7 with a third photodetector 7. The plurality of photodetectors 7 comprise preferably at least four photodetectors 7 with a fourth photodetector 7. The plurality of photodetectors 7 comprise preferably at least five photodetectors 7 with a fifth photodetector 7.

The light emitting unit(s) 6 and the photodetector(s) 7 are arranged such that the light emitted by the light emitting unit 6 is not directly measured by the photodetector(s) 7 (at least when the tissue is in contact with the tissue contact surface 2 so that for the PPG measurement only the light from the tissue is detected). This can be achieved by non transmissive means or light blocking means between the light emitting unit(s) 6 and the photodetector(s) 7 (especially, when they are arranged in the same plane/tissue contact surface 2). This improves the measurement quality but is not essential for the invention.

The plurality of light emitting units 6 and/or the plurality of photodetectors 7 are preferably arranged such that the plurality of light emitting units 6 and/or the plurality of photodetectors 7 are well distributed over the tissue contact surface 2 (or at least the portion of the surface 2 dedicated for the PPG measurement). In a preferred embodiment, the light emitting units 6 and the photodetectors 7 are arranged such that they alternate each other. This means that (in at least one direction, preferably in two directions), a first photodetector 7 is arranged next to a first light emitting unit 6 which is again arranged next to second photodetector 7 which is again arranged next to second light emitting unit 6 and so on. Thus, each photodetector 6 is arranged between at least two light emitting units 6 (if not arranged at the outer side of the PPG sensor field), and/or each light emitting unit 6 is arranged between at least two (preferably four) photodetectors 7 (if not arranged at the outer side of the PPG sensor field). Preferably, the distances of neighboring photodetectors 7 and light emitting units 6 are (always or in more than 50%) the same. However, they could also have different distances.

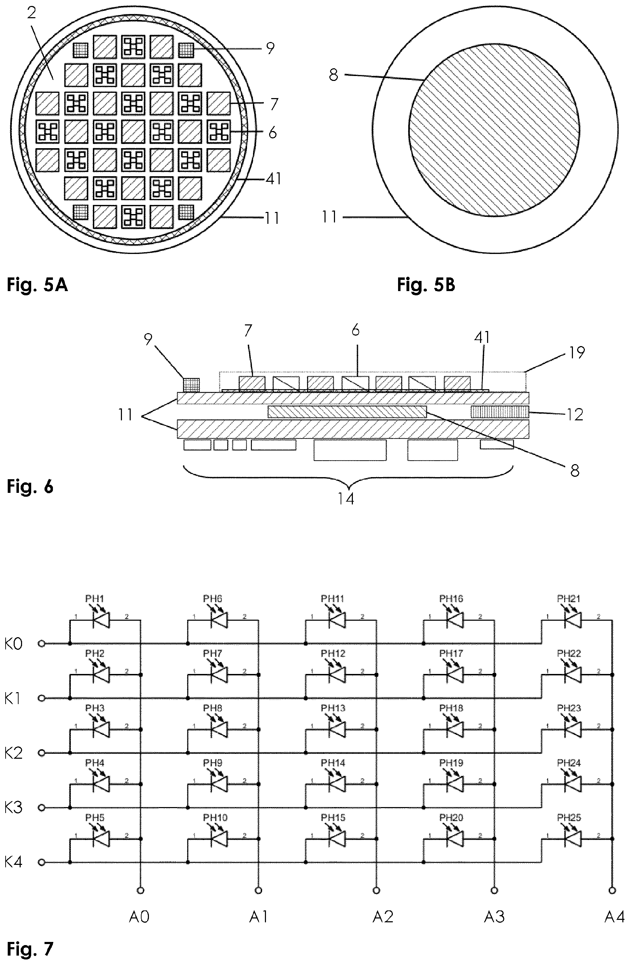

In a preferred embodiment, the plurality of light emitting units 6 and/or the plurality of photodetectors 7 are arranged in a matrix/grid arrangement in rows and columns. Preferably, a row and/or column arrange a light emitting unit 6 and a photodetector 7 in an alternating way. Thus, a photodetector 7 is surrounded by two light emitting units 6 in the same row or column or by four light emitting units 6 in the same row and column. Equally, light emitting unit 6 is surrounded by photodetectors 7 in the same row or column or by four photodetectors 7 in the same row and column. Such an arrangement allows a very flexible way of PPG measurement as nearly any couple/pair of one of the light emitting units 6 and one of the photodetectors 7 can be used to measure the PPG signal. Due to the matrix arrangement, the optimal optical path can be chosen with different distances, different locations and other different parameters like spectral characteristics. Fig. 2A shows a first matrix arrangement in which each row and column has the same number of PPG items 6, 7 (allowing a rectangular matrix arrangement). To be noted that the number of photodetectors 7 or light emitting units 6 change from row to row, if the number of PPG items 6, 7 per row is odd. The same holds obviously for the columns. Fig. 5A shows another matrix arrangement of the PPG sensor 3 in which the PPG the number of items 6, 7 in different rows and/or columns distinguish. In this case, the matrix arrangement has roughly the shape of a circle.

The PPG sensor 3 comprises further a signal unit for providing or outputting a PPG signal of the tissue based on the light detected at the photodetector. In this case, the PPG sensor 3 provides a plurality of optical paths (provided by a plurality of light emitting units, a plurality of photodetectors, a plurality of light emitters, different light intensities, etc.), the signal unit is configured to provide or output one PPG signal measured for each optical path. The PPG signal(s) are preferably output to a processing means 5 for further processing the PPG signal(s).

The signal unit comprises preferably a driving section for driving the light emission of the light emitter unit(s) 6 and an acquisition section for measuring the light detected at the photodetector(s) 7. The light emitted from one of the light emitter units 6 and detected by one of the photodetectors 7 provides one optical path which results in its own PPG signal, when this optical path is measured. Obviously, each combination of light emitter unit 6 and photodetector 7 can have even multiple optical paths and thus PPG signals, e.g. for multiple emitters 61, 62, 63, 64, 65 or for multiple light intensities. By measuring each optical path of each combination of light emitting unit 6 and photodetector 7, many different PPG signals can be measured and output by the signal unit.

The acquisition section provides preferably a common signal acquisition electronics for the plurality of photodetectors 7. This reduces the complexity of the electronics of the PPG sensor 3. Preferably, the selected photodetector 7 whose PPG or other signal shall be measured is switched/connected to the common signal acquisition electronics of the PPG sensor. The matrix arrangement allows to connect the photodetectors 7 of the same row with a common row conductor K, and/or to connect the photodetectors 7 of the same column with a common column conductor A. Thus, the photodetectors 7 of different columns 0, 1, 2, 3, 4 are connected by different column connectors A0, A1, A2, A3, A4. Equally, the photodetectors 7 of different rows 0, 1, 2, 3, 4 are connected by different row connectors K0, K1, K2, K3, K4. By switching a couple/pair of a column connectors A0, A1, A2, A3, A4 and a row connectors K0, K1, K2, K3, K4 to the common acquisition electronics, the PPG signal of a selected photodetector 7 can be measured with the common acquisition electronics. The PPG signals of the photodetectors 7 must obviously be measured one after the other. But since for most applications only few optical channels are required, this is normally enough. It is also possible to measure a composite signal of the light detected at a plurality of photodetectors, e.g. of one row. Fig. 7 shows such a photodetector array.

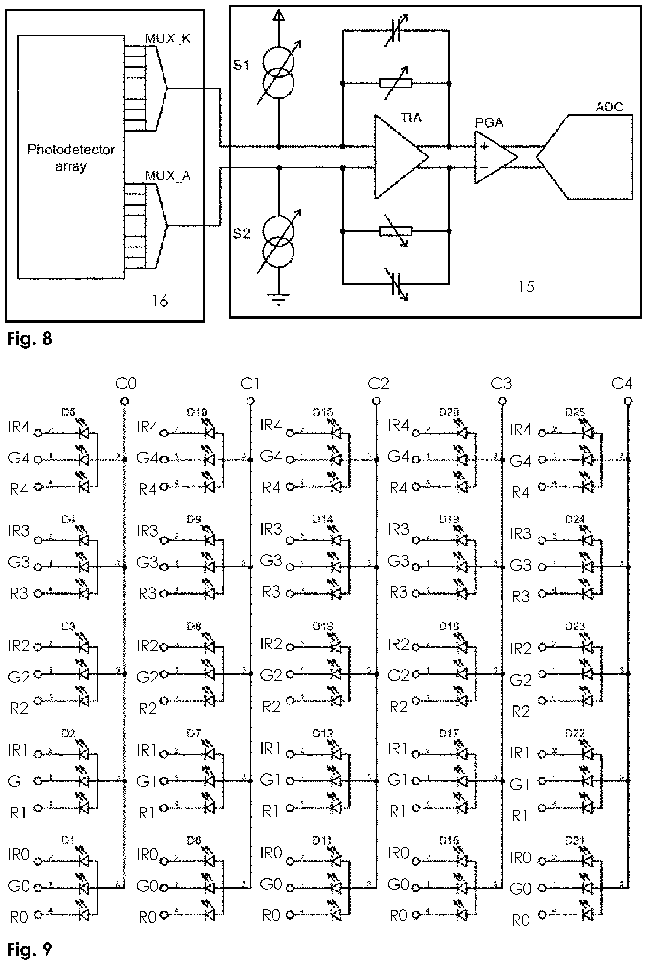

Fig. 8 shows an exemplary embodiment for such a common acquisition section 15 and a switching means 16 which allows to switch one or more row conductors K and one or more column conductors A to the common acquisition electronics 15 to measure the light detected at the photodetectors connected with its respective row and column conductor A and K to the common acquisition electronics 15. Preferably, there are at least two, preferably at least three, preferably at least four row (detector) conductors. Preferably, there are at least two, preferably at least three, preferably at least four column (detector) conductors. The common acquisition electronics 15 is realized as a Transimpedance amplifier (TIA). The TIA transforms the current flowing between the row conductors and the column conductors connected over the respective photodetectors 7 into a respective voltage. Such a configuration also allows the parallel connection of photodetectors 7 to increase the detection area and the amplitude of the optical signal. In this case, care must be taken so that the acquisition bandwidth is not affected by the consequent cumulative increase in stray capacitance at TIA input. The obtained voltage can be amplified by a programmable gain amplifier (PGA) before being digitized by an analog to digital converter (ADC), whose output value is used to process the corresponding optical information. To take full advantage of the dynamic range of the ADC converter, both the PPG DC component and the offset value induced by the ambient light influence are actively removed by the programmable constant current sources S1 and S2. It is understood that other common acquisition electronics 15 and circuits elements combinations can be used.

In one embodiment, the PPG sensor 3 comprises a common driver section (not shown) which is used to drive the different light emitting units 6 independently from each other. If the light emitting units 6 have different emitters 61, 62, 63, 64, 65, preferable each of those can be controlled independently from the others. The common driver electronics can be switched by an emitter switching means to the respective light emitting unit 6 or emitter 61, 62, 63, 64, 65 which shall emit light. Fig. 9 shows an embodiment for connecting 25 light emitting units 6 (D1 to D25). In a preferred embodiment, the PPG sensor 3 comprises a plurality of row emitter conductors IR, G, R and a plurality of column emitter conductors C. Preferably, there are at least two, preferably at least three, preferably at least four row emitter conductors. Preferably, there are at least two, preferably at least three, preferably at least four column emitter conductors. The plurality of light emitting units 6 comprises a plurality of column sub-groups of light emitting units 6 with each column sub-group connected by a different column emitter conductor C0, C1, C2, C3, C4. The plurality of light emitting units comprises a plurality of row sub-groups of light emitting units 6 with each row sub-group connected by a different row emitter conductor. The emitter switching means is configured to switch one of the row emitter conductors and one of the column detector conductors C0, C1, C2, C3, C4 to the driver electronics to provide a drive current to the intersecting light emitting unit 6 being in the row sub-group connected by the row emitter conductor through the emitter switching means to the driver electronics and being in the column sub-group connected by the column emitter conductor C0, C1, C2, C3, C4 through the emitter switching means to the driver electronics. In case, the light emitting units 6 comprise a plurality of separately controllable emitters 61, 62, 63, 64, 65, in Fig. 9 three, one of the row or column emitter conductors comprises a plurality of sub-conductors for connecting each emitter of the light emitting unit 6 with a different sub-conductor. Such an arrangement allows to use the same driver for different light emitting units 6 and/or emitters 61, 62, 63, 64, 65.

The light emitter units 6 and the photodetectors 7 are preferably arranged on a substrate 11, e.g. a PCB substrate 11. The substrate 11 plane provides the plane of the tissue contact surface. The PPG items 6, 7 are arranged on the substrate 11 and can be connected to the diver electronics or the signal acquisition electronics 15 through conductor tracks (and maybe through switching means as explained above). The electronics 14 comprising the driver and signal acquisition electronics 15 could be arranged on the other side of the substrate 11 or on a second substrate layer 11 as shown in the embodiment of Fig. 6 in which the device comprises a sandwich of two substrate layers 11. In such a sandwich structure of two substrates 11, the force sensor 8 could be arranged between the two substrates 11. In the shown embodiment, the PPG device comprises further a communication module 12 for communicating with other devices. The communication module 12 is configured for example to output PPG signals and/or processed parameter(s) and/or to receive requests for measuring a certain processed parameter.

The light emitting units 6 and photodetectors 7 are preferably protected (from moisture, sweat, dust, and other contaminants) by a transparent (for the wavelengths range of the device) protective cover placed over them. The protective cover can be made from a rigid, flexible, or semi-flexible biocompatible material coated with an antireflective paint, enamel, coating, or polymer. At the same time, to improve the signal-to-noise ratio, an opaque light barrier is placed between the photodetectors and light emitters to prevent direct light transfer between them. This can be integrated into the protective cover or be made of some suitable material, such as opaque epoxy resin. In addition, the same optical elements of the reflective optical sensor system can include such protection in their package. Depending on the configuration, optical filters "tuned" to one or more specific wavelengths may be used. In addition, the protective cover will allow the coupling between the tissue and the capacitive sensor, as well as the thermal transfer with any temperature sensor 9 configured to determine the tissue thermal properties.

The PPG sensor 3 is preferably a reflective PPG sensor 3 with the light emitting units 6 and the photodetectors 7 being arranged in the same plane, preferably on the same substrate 11. However, the PPG sensor 3 described above could also be applied in transmissive PPG sensor 3 with a first tissue contact sub-surface and a second tissue contact sub-surface arranged on two sides of a tissue, e.g. a finger, toe, ear, etc. Each sub-surface could comprise a plurality of light emitter units 6 and/or a plurality of photodetectors 7. In one embodiment, all photodetectors 7 are arranged in the first sub-surface while all light emitter units 6 are arranged on the second sub-surface. In another embodiment, one or both sub-surfaces comprise a combination of photodetectors 7 and light emitter units 6 allowing to have a reflective and transmissive measurement in the same PPG sensor.

The PPG sensor 3 measures at least one optical path (also called optical channel) between (a light emitter 61, 62, 63, 64, 65 of) a light emitting unit 6 and a photodetector 7.

The fundamental working principle of PPG can be explained by the modified Beer-Lambert law. In PPG, a volume of perfused tissue is interrogated with light at a target wavelength throughout each cardiac cycle. With volumetric change in pulsatile tissue components between systole and diastole, the absorbance of light within the tissue also varies with time, leading to a continuous PPG wave. In reflective PPG, photons do not usually travel directly from the transmitter 6 to detector 7 but can be scattered in all directions. The scattering character in the biological tissues can be described by Henyey-Greenstein's phase function $P_{ph}$:

$$P_{ph}(\vartheta) = \frac{1 - g^2}{4\pi(1 + g^2 - 2g * cos\vartheta)^{3/2}}$$

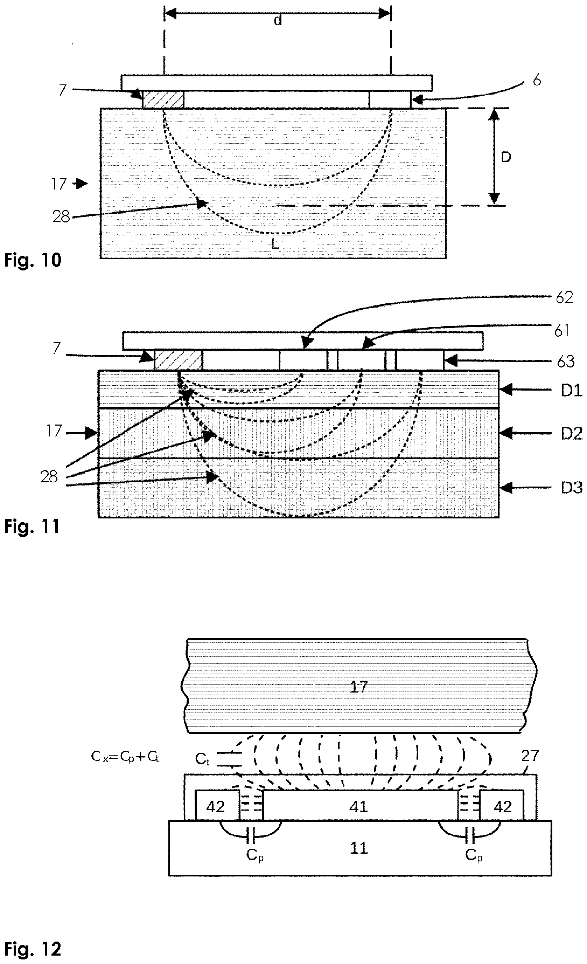

where g is an anisotropy factor. Due to the high scattering properties of light in tissues, the photons from the light sources travel across the tissue forming an arch-shaped pathway, also known as banana shape, before returning to the photodetector 7 as shown in Fig. 10. The reflective length L of the arc 28 is greater than the geometrical distance d between the light emitter (unit) 6 -photodetector 7 pair (emitter-detector pair) and the mean penetration depth D in the tissue (vessel depth). An optical channel or path is defined as the combination of any light emitter (unit) 6, 61, 62, 63, 64, 65 with any photodetector 7, forming an emitter-detector pair in which photons travel in tissue 17 via an optical pathway. The term optical path and optical channel are used as equivalents in here.

In PPG measurements, the depth D of light penetration also depends on the wavelength of the light used to illuminate the tissue 17. At wavelengths with low absorption by water (between 400 and 1100 nm), as the wavelength increases, the phenomenon of dispersion in the dermis decreases, which is why the penetration capacity of said wavelength increases. This phenomenon is exemplified in Fig. 11, which shows the penetrating strength of optical channels 28 of three wavelengths provided by three different emitters 61, 62, 63, which reach the vascular depths D1, D2 and D3, respectively.

The PPG sensor 3 provides a plurality of optical paths (or channels) due to the plurality of light emitting units 6 and/or the plurality of photodetectors 7. The PPG sensor 3 can measure the PPG signal with each of the plurality of optical paths. Theoretical optical paths which cannot be measured by the (signal unit of the) PPG sensor 3 or the device 1 shall not be considered as optical paths as meant herein.

In a preferred embodiment, the plurality of optical paths comprises preferably a first optical path from the first light emitter unit 6 to the first photodetector 7, a second optical path from the first light emitter unit 6 to the second photodetector 7 and a third optical path from the first light emitter unit 6 to the third photodetector 7. In a preferred embodiment, the plurality of optical paths comprises preferably for each light emitting unit 6 an optical path to at least three different photodetectors 7. The plurality of optical paths comprises preferably each possible emitter-detector couple between the light emitting units 6 and the photodetectors 7. Thus, an embodiment with N light emitting units 6 and M photodetectors 7 provides at least M times N possible optical paths. The plurality of optical paths comprises preferably each possible emitter-detector couple between the light emitters (sub-units) 61, 62, 63, 64, 65 and the photodetectors 7. If the light emitting units 6 comprise different light emitters 61, 62, 63, 64, 65 with L different spectral characteristics, the plurality of optical paths comprises even M times N times L different optical paths.

The plurality of optical paths comprise preferably for each (measurable) emitter-detector couple between the light emitting units 6 or the light emitters 61, 62, 63, 64, 65 and the photodetectors 7 or for each of the three emitter-detector couples between the first emitting unit 6 and the first, second and third photodetector 7 a first intensity optical path realized with a first light intensity and a second intensity optical path realized with a second light intensity (for the same emitter-detector couple). The first intensity optical path for an emitter-detector couple is realized by emitting the light with the first light intensity of the light emitter (sub-) unit of the emitter-detector couple and measuring the PPG signal at the photodetector of the emitter-detector couple. The second intensity optical path for an emitter-detector couple is realized by emitting the light with the second light intensity of the light emitter (sub-) unit of the emitter-detector couple and measuring the PPG signal at the photodetector of the emitter-detector couple. The first light intensity and the second light intensity have different intensities. Analogously, the plurality of optical paths could comprise for each emitter-detector couple further a third intensity optical path realized with a third light intensity and even more light intensity paths.

The plurality of optical paths could comprise further optical paths realized by combining at least two optical paths. For certain measurements, it could make sense to emit light of two or more different wavelengths or of different locations during the same measurement.

The PPG sensor 3 allows to measure all or multiple of the plurality of optical paths and to select based on the measurement signal of the different optical paths the best optical path(s). This can be realized based on a quality (index) parameter determined from the different measurements or PPG signals. The quality (index) parameter can be a pulse quality index (PQI) or signal quality index (SQI) like the perfusion index, the skewness, kurtosis, entropy, zero crossing rate, signal to noise ratio (SNR). The different optical paths allow further to select different optical paths for the calculation of different parameters from the PPG signal. So, based on the (health) parameter, which is desired, a different optical path can be chosen. This different optical path could be chosen among the plurality of optical paths or the best optical paths. This will be described in more detail below.

In a preferred embodiment, the device 1 comprises further a capacitive sensor 4. The capacitive sensor 4 is configured to measure a capacitive change due to a contact and/or vicinity of the tissue 17 to the tissue contact surface 2. These capacitive sensors 4 are also called capacitive touch/contact sensors. The capacitive sensor 4 is arranged in the tissue contact surface 2. The capacitive sensor 4 comprises at least one capacitive electrode 41 arranged in the tissue contact surface 2.

Many capacitive sensors 4 measure such a capacitive change by measuring the signal (current or voltage) between two electrodes arranged in the tissue contact surface 2 and not having a galvanic connection. Thus, the two electrodes form a capacitor with a capacitance value. The vicinity of a tissue 17 close to the two electrodes changes the capacitance value between the two electrodes and this change of capacitance can be detected in the measured signal between the two electrodes. For example, each touch pixel of a capacitive touch sensors of touch screens is normally realized like this. However, such a solution requires a lot of space in the tissue contact surface 2 and requires often transparent electrodes which are complex and expensive.

In the preferred solution, a very simple capacitive sensor 4 is realized by applying a signal to the capacitive electrode 41 and measuring the signal at the same capacitive electrode 41, i.e. without a capacitor between a drive signal and a measurement signal. The capacitive electrode 41 has a certain parasitic capacitance value Cp as schematically shown in Fig. 12. When a tissue 17 comes in the vicinity or in contact with the tissue contact surface 2, the additional tissue capacitance value Ct is added to the constant capacitance value Cp of the capacitive electrode 41. Thus, the capacitance value Cx of the capacitive electrode 41 is the sum of the constant capacitance value Cp and the capacitance value Ct whose value depends on the vicinity, pressure and/or size of the tissue 17. Thus, the signal measured at the capacitive electrode 41 differs from the drive signal depending on the capacitance value Cx, i.e. depending on the capacitance value Ct

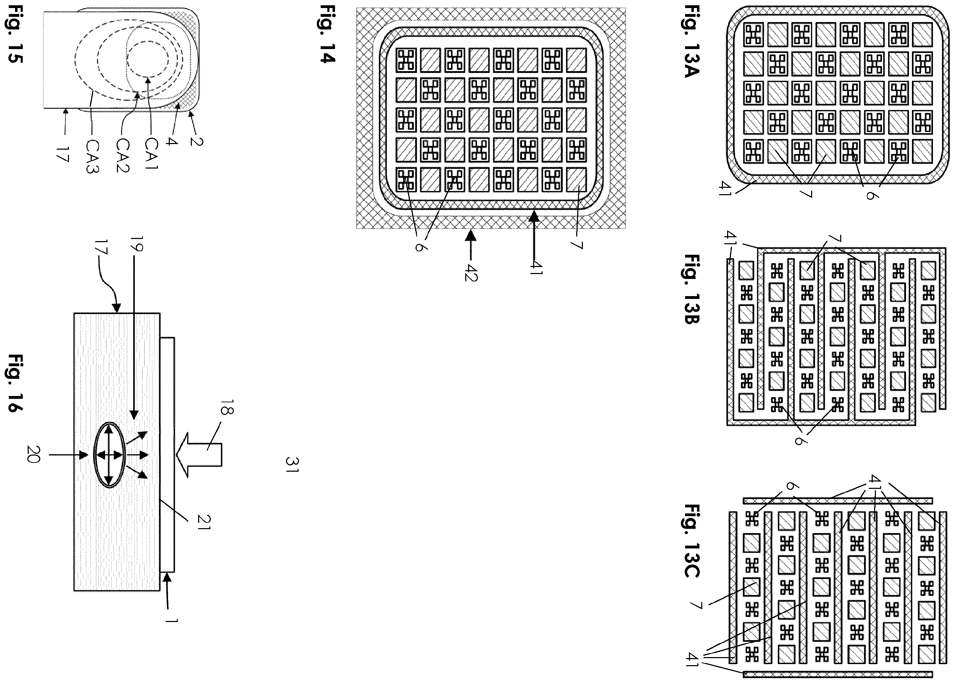

Preferably, the capacitive electrode 41 is arranged in the same substrate 11 as the PPG sensor 3 or its PPG items 6, 7. But it is obviously also possible to arrange the capacitive electrode 41 in another substrate 11. Different shapes of the capacitive electrode 41 are possible. In one embodiment as for example shown in 13A; Fig.2A and 5A, the capacitive electrode 41 surrounds the PPG sensor 3 or the plurality of PPG items 6 and 7. This has the advantage that capacitive sensor 4 and the PPG sensor 3 can work together, when the tissue 17 is placed on the tissue contact surface 2. In this embodiment, the capacitive electrode has a closed ring shape. Obviously, other shapes of the capacitive electrode 41 are possible. The sensitivity can for example be increased by increasing the surface of the capacitive electrode 41 on the tissue contact surface 2. This can be achieved by extending the electrode further between the rows and/or columns of the PPG items 6,7 of the PPG sensor 3. However, this reduces the space available for the PPG items 6, 7. Fig. 13B and 13C show further embodiments with multiple capacitive electrodes 41 and with different shapes. To have the capacitive electrode(s) extend parallel to each row or column of the PPG items 6,7 increases the surface of the capacitive electrode 41 and/or distributes the presence of the electrode 41 over the tissue contact surface 2, especially in the region of the PPG sensor 3 without the necessity to make the electrodes transparent. In Fig. 13B, two distinct capacitive electrodes 41 extend from two opposed sides into the tissue contact surface 2. In this embodiment, the two capacitive electrodes are arranged like forks which engage into each other. Preferably, the two capacitive electrodes have the same size. Preferably, for each capacitive electrode 41, an independent capacitive value is measured. By comparing the two values, a rough tissue position (with respect to the two opposed sides) can be calculated based on the two capacitive values. If the two values are equal, a symmetric tissue 17 is positioned in the center between the opposed sides. If one value of one capacitive electrode 41 is much bigger than the other one, the tissue 17 is positioned closer to this side of the electrode 41 with the larger value. Fig. 13C shows a similar solution as in Fig. 13B, but with even more independently measurable capacitive electrodes 41. Based on the capacitive value of the different capacitive electrodes 41, the position of the tissue 17 on the tissue contact surface 2 can be determined with even higher precision. The horizontal capacitive electrodes 41 arranged between the rows of the PPG items 6, 7 allow to determine position of the tissue 17 in the direction perpendicular to the row direction and/or to the extension of these electrodes 41. The two vertical capacitive electrodes 41 are arranged at the two sides of the PPG sensor 3 which are perpendicular to the horizontal capacitive electrodes 41 and/or to the rows. These vertical capacitive electrodes 41 allow to determine the position of the tissue 17 on the tissue contact surface 2 between these two vertical capacitive electrodes 41. However, the higher precision in the tissue 17 location goes on the costs of a lower sensitivity of the capacitive electrodes 41 as their surfaces are smaller.

A further improvement of the capacitive sensor 4 is a shielding electrode 42, e.g. the hatched electrode in Fig. 14. The shielding electrode 42 is arranged around the capacitive electrode 41. It has the purpose to shield the capacitive electrode 41 from other capacitive effects which are not constant to reduce the influence of the capacitive disturbances on the measurement.

Especially the combination of the PPG sensor 3 and the capacitive sensor 4 can achieve very interesting measurements as explained later, especially when combining multiple photodetectors 7 of the PPG sensor 3 with the capacitive sensor 4.

The capacitive sensor 4 can be used for one or more of the following purposes (when the tissue 17 is in contact with the tissue contact surface 2): determine contact area of tissue 17 on the contact surface 2, determine contact force (calibration required), determine tonometric signal, determine respiration signal, movement artifact detection, determine elastic and viscoelastic tissue behaviour. The capacitive sensor 4 can however also be used as a proximity sensor to detect the distance of the tissue 17 to the tissue contact surface 2. Details of how these measures can be computed will be described below. The capacitive sensor 4 provides or outputs the capacitive value of the one or more capacitive electrodes 41 of the sensor 4, preferably to the processing means 5.

The device 1 could use further one or more of the following auxiliary sensors to further improve its performance.

The contact force sensor 8 is principally configured to determine the contact force between the tissue contact surface 2 and the tissue 17. The contact force sensor 8 is preferably arranged such that the central axis of the force sensor corresponds to the central axis of the set of PPG items 6, 7 of the PPG sensor 3 and/or corresponds to the normal vector of the tissue contact surface 2. Depending on the type of contact force sensor 8, the contact force sensor is arranged in the tissue contact surface 2 or below. Various types of force sensors can be selected, which can be resistive, capacitive, piezoresistive, mechanical, load cell, strain gauge or membrane potentiometer. Piezoresistive and capacitive sensors are the ones that provide the best results in terms of sensitivity, repeatability, hysteresis, and accuracy. The contact force sensor 8 can be further used to determine the tonometric signal. The force sensor 8 can work complementary to the capacitive sensor 4. The capacitive sensor 4, while being very sensitive to pressure variations, it can lack accuracy that can be provided by the force sensor 8. The combination of both sensors can be used to accurately extract the skin visco-elastic properties, to improve contact force determination and/or to improve the determination of the tonometric signal.

The temperature sensor 9 is configured to measure the temperature of the tissue 17. Temperature affects the perfusion of peripheral blood vessels, so it can be used for proper normalization of the multiwavelength PPG signal waveform before it is used to determine physiological parameters. The temperature sensor 9 is preferably arranged in the tissue contact surface 2. An additional temperature sensor can be used to determine the ambient temperature and assess the impact of this temperature on changes in the PPG signal induced by thermal stress on the human/animal body. This sensor can be combined with the temperature sensor 9.

The inertial sensor 10 is configured to detect the movement of the tissue 17, i.e. of the user. The inertial sensor 10 comprises preferably an accelerometer and/or a gyroscope. This can be used for processing and filtering of the movement artifacts in the PPG signals. It can further be used for detecting user activity and control the timing of the PPG and other sensor measurements.

The processing means 5 is preferably used to process the measurement(s) from one or more of the PPG sensor 3, the capacitive sensor 4, the force sensor 8, the temperature sensor 9 and the inertial sensor 10. The processing means 5 is preferably also used to control the different sensors, i.e. to trigger their measurements. The functions of the processing means 5 can be realized by one or more processing unit(s). The processing means 5 can comprise for example a microprocessor, microcontroller, a Complex Programmable Logic Device (CPLD), a Field Programmable Gate Array (FPGA), a System on a Chip (SoC) or an Application-Specific Integrated Circuit (ASIC). The processing means 5 is preferably fully integrated in the device 1. However, it is also possible that the processing means 5 or a part of it is not arranged in the device 1. E.g. some or all functions described subsequently can be arranged in a connected device. For example, the device 1 could be a sensor device 1 which is connected over a Bluetooth connection or other communication connection to a smartphone which is used as processing means 5.

The processing means 5 is preferably configured to perform a signal processing. The processing means 5 is configured or the signal processing comprises to process the PPG signal(s) of the PPG sensor 3 in order to determine at least one processed parameter. The at least one processed parameter is preferably output by an output means 27 described later. The processed parameter is preferably an indicator about the health, fitness or body of the user/tissue 17. The processed parameter is preferably a cardiac-, blood- and/or respiration related parameter. The at least one processed parameter comprises preferably one or more of heart rate, pulse rate variability, respiratory rate, SpO2, blood pressure, peripheral arterial disease (PAD) detection, .... The processed parameter is preferably computed periodically/continuously. The processed parameter is preferably computed in each period or sample based on the PPG signal(s) of a predetermined length at the time of the sample. The predetermined length can be different for different processed parameters. The predetermined length is preferably longer than one heartbeat, preferably longer than a plurality of heart beats. In a preferred embodiment, the device 1 is configured to determine different processed parameters based on the PPG signals. Preferably, these different processed parameters use different PPG signals, i.e. different optical paths for obtaining the different processed parameters. In one embodiment, the PPG device 1 determines all different processed parameters in a measurement cycle. In a different embodiment, the PPG device 1 has different measurement modes for measuring in each measurement mode at least one different processed parameter.

In a preferred embodiment, the PPG device 1 is configured to be calibrated. In a preferred embodiment, the PPG device 1 is calibrated for a specific tissue 17 before performing the PPG measurement (signal processing) and/or during the signal processing to re-calibrate the device. A re-calibration is preferably done when/triggered by a calibration indicator indicating the necessity to re-calibrate. The calibration indicator can be based on the signal, e.g. a quality indicator of the PPG signal(s) as described below and/or a change in the auxiliary measurements of the auxiliary sensor, wherein the auxiliary sensor comprises one or more of the sensors 4, 8, 9, 10. The calibration indicator can be different for different types of calibration. The re-calibration could however also be triggered/done periodically (i.e. without a calibration indicator). However, a calibration indicator has the advantage that processed parameter determined in the signal processing mode has a high reliability, because once the calibration indicator indicates the necessity of a re-calibration, the PPG device 1 is re-calibrated so that the PPG measurement remains reliable. The re-calibration can be also used only with some or one of the subsequently described calibrations. The PPG device 1 could also be used without such a calibration. Before describing different possible calibrations, which can be done, some parameters which might be used for the calibration or signal processing mode of the PPG device 1 are described subsequently and which are provided by the processing means 5 based on one or more of the sensors 3, 4, 8, 9, 10.

The processing means 5 might be configured to detect, if a tissue 17 is in contact with the tissue contact surface 2 or not. This can be determined based on the capacitive sensor 4 and/or based on the contact force sensor 8 and/or based on the PPG sensor 3. Most preferably, the tissue contact is detected based on the capacitive sensor 4, eventually In combination with the PPG sensor 3 and/or the contact force sensor 8. This could be used to wake up the device 1 from sleep mode into an active mode. This could also be used to start a measurement or a scanning/selection of the optical paths.

The processing means 5 might be configured to determine the location of tissue and/or estimate the contact area of the tissue 17 on the tissue contact surface 2 based on the capacitive sensor 4. For this function, it is preferred that the capacitive sensor 4 has two or more capacitive electrodes 41. However, the size of the contact area could also be determined with only one capacitive electrode 41.

The processing means 5 might be configured to determine the contact force of the tissue 17 with which it is pushing against the tissue contact surface 2. This can be done based on the contact force sensor 8 and/or based on the capacitive sensor 4. In the latter case, a calibration of the capacitive sensor 4 is necessary. Such a calibration of the capacitive sensor for measuring a contact force is well known and is referred to the literature. The calibration must be done once before the first operation of the device 1 or when the necessity for a recalibration of the device is detected.

The processing means 5 might be configured to determine moments of movement of the tissue 17 during the measurement. This allows to detect movement artifacts in the other measurements of the PPG sensor 3, the capacitive sensor 4 and/or the force sensor 8. The moments of movement of the tissue 17 can be detected based on the capacitive sensor 4, the pressure sensor 8 and/or the inertial sensor 10. These moments of movement detected, can be used to remove movement artifacts in the mentioned measured signals like the PPG signal(s), the capacitive signal and/or the contact force signal. Instead of removing the artifacts, it could also be used to determine the signal portions or time portions of the signal(s) to be used for further processing which do not comprise such moments of movement.

The processing means 5 might be configured to determine the elastic and viscoelastic tissue behaviour of the tissue 17. This can be used to perform the PPG measurements (and other measurements) with the correct contact pressure 18 exerted by the tissue 17 on the tissue contact surface 2.

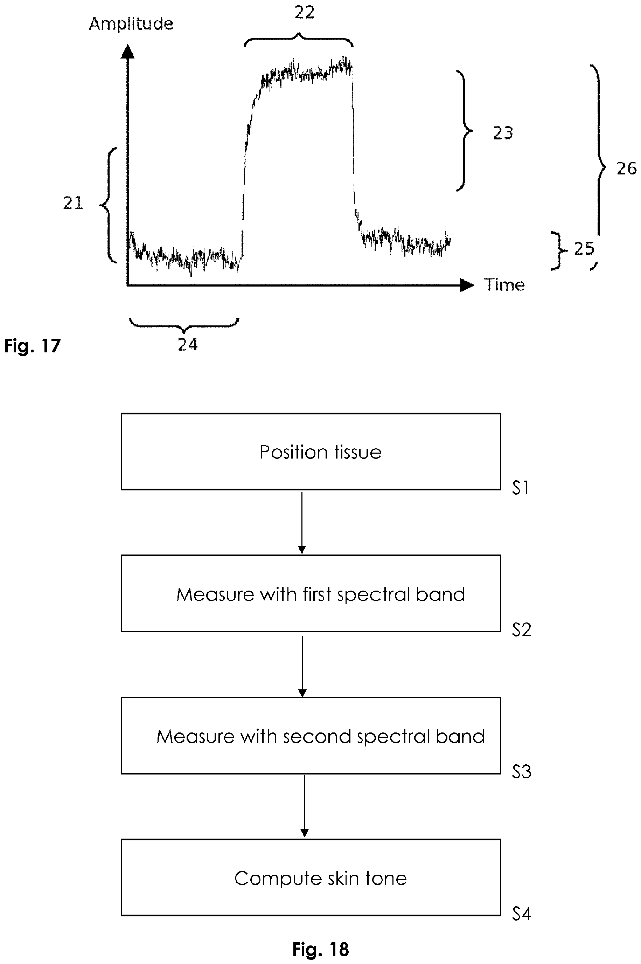

The elastic and viscoelastic tissue behaviour can be determined. This can be done based on the capacitive signal from the capacitive sensor 4 and/or based on the contact pressure sensor 8. Preferably, the capacitive sensor 4 is used to detect the elastic and viscoelastic tissue behaviour, eventually in combination with the contact force sensor 8. Fig. 17 shows the capacitive signal of the capacitive sensor 4 acquired for different contact pressures 18 of the tissue 17. It can be seen that there are four zones corresponding to the elastic and viscoelastic tissue behavior. As the tissue sits on the tissue contact surface 2 exerting positive pressure on it, two zones appear. The elastic zone 21 has a linear relationship between the tissue deformation and the sensor capacitance, while in the viscoelastic zone 22, when the positive pressure is reaching its maximum, the behavior has a non-linear relationship. When negative pressure is exerted, the retraction zone 23 appears, and in this zone its behavior is mostly elastic. A residual zone 25 appears as a result of the plastic deformation suffered by the tissue 17, the negative pressure is going to its minimum. The baseline 24 corresponds to the capacitive/force sensor output when the tissue 17 is not on the tissue contact surface 2 of the PPG device 1. The set of the four previous zones 21, 22, 23, and 25 forms the tissue flexibility zone 26.

The processing means 5 is further configured to determine the distance of the tissue 17 to the tissue contact surface 2 before it contacts this surface 2. This is preferably realized by the capacitive sensor 4. If even used in PPG devices, this distance measurement is normally realized by an optical measurement, mainly with infrared light. However, such an optical distance measurement requires a higher power consumption. Therefore, the capacitive distance measurement is highly preferred. Preferably, the configuration of the capacitive sensor 4 is changed for the distance measurement (compared to the other measurements done when the tissue 17 is in contact with the tissue contact surface 2). Normally, the sensitivity of the capacitive sensor 4 is increased. However, the distance measurement could also be performed with the same parametrization. This distance of the tissue 17 is mainly used for the skin tone calibration explained later. This mode could further be used to detect the vicinity of a tissue 17 to switch the device 1 from a sleep mode in an active mode (instead of the tissue contact detection). Even if the capacitive distance measurement is preferred, it is also possible to perform the distance measurement optically by the PPG sensor 1. In one embodiment, the distance is determined based on a combination of the capacitive sensor 4 and the PPG sensor 3 to increase the precision of the distance measurement.

The processing means 5 is further configured to determine the skin tone of the tissue 17. This can be done based on the PPG sensor 3. The PPG sensor 3 can be used/configured to work as a skin tone sensor. The principal light absorbers (chromophores) in the skin are melanin and hemoglobin. The skin color is derived from the melanin index (MI), which is a measure of the melanin content in the skin, responsible for skin pigmentation. The measurement is based on the diffuse remittance/remission spectrometry principle whereby light is directed onto the skin and the diffuse remittance/remission, i.e., the reflection or back scattering of light by the tissue, is measured. The diffused light has passed into the upper region of the skin and it has been scattered and absorbed by the cutaneous structure and pigments, including melanin. The light absorption of melanin differs over different wavelengths much less than the light absorption of hemoglobin. These absorbance differences are used to calculate the melanin index based on first spectral band, for example red light and based on a second spectral band being with wavelengths being higher than the first spectral band, for example infrared light. These absorbance differences can be used to calculate the melanin index, which is a relation between the measured reflectance results in terms of a reflectance index of the first spectral band and the light of the second spectral band. The skin tone could be used with other spectral bands and/or with more than two spectral bands.

Fig. 18 illustrates the main steps of this measurement to determine the skin tone according to a first embodiment. In a first step S1, the tissue 17 is positioned with a (non-zero) distance over the tissue contact surface 2. Preferably, the tissue 17 is positioned in a distance larger than 1 mm, preferably than 3 mm, preferably than 4 mm, preferably than 5 mm. Preferably, the tissue 17 is positioned in a distance smaller than 50 mm, preferably than 40 mm, preferably than 30 mm, preferably than 25 mm, preferably than 20 mm. The positioning process can be completely manual, e.g. described in some user manual. However, the positioning process can also be supported by the PPG device 1 e.g. by outputting on a display or via an audio means as a support output indicating the preferred distance of the tissue to the tissue contact surface 2. Even more preferably, the PPG device 1 could measure the distance of the tissue 17 as described above in order to help the user to position his tissue 17 in the correct distance. This measured distance could also be used to trigger the start of the skin tone measurement in steps S2 and S3, once the tissue 17 is positioned correctly. Thus, steps S2 and S3 are performed, while the tissue 17 is in this position of S1. In step S2, the tissue 17 is illuminated with the light of the first spectral wavelength, here red. This illumination can be done by using one or more light emitting units 6. Preferably, a plurality of light emitting units 6, preferably all are used to increase the light intensity for the measurement. Preferably, only the light of the light emitter 61, 62, 63, 64, 65 of the respective first spectral band is used. The emitted light is reflected from the tissue 17 and measured by one or more photodetectors 7 of the PPG sensor 3 as a first reflected tone light. Preferably, a plurality of photodetectors 7, preferably all are used to detect the first reflected tone light. In step S3, the tissue 17 is illuminated with the light of the second spectral wavelength, here infrared. This illumination can be done by using one or more light emitting units 6. Preferably, a plurality of light emitting units 6, preferably all are used to increase the light intensity for the measurement. Preferably, the same number of light emitting units 6 as for step S2 are used. Preferably, only the light of the light emitter 61, 62, 63, 64, 65 of the respective second spectral band is used. The emitted light is reflected from the tissue 17 and measured by one or more photodetector 7 of the PPG sensor 3 as a second reflected tone light. Preferably, a plurality of photodetectors 7, preferably all are used to detect the second reflected tone light. Preferably, the same number of photodetectors 7 as for step S2 are used. The steps S2 and S3 are performed one after the other (at any order), i.e. not contemporaneously. In step S4, the skin tone is computed based on the first reflected tone light and based on the second reflected tone light. Preferably, the skin tone is computed based on the melanin index. The melanin index can be computed according to the following formula

$$M_I = \left(\frac{500}{\log 5}\right) * \left(\log\left(\frac{IR}{R}\right) + \log 5\right)$$

where IR is the second reflected light and R is the first reflected light. The MI is expressed in arbitrary units from 0 to 1000, being 0 the lightest and 1000 the darkest skin color. The MI value is correlated with the Fitzpatrick scale stored in a look-up table. The skin tone can be a processed parameter which is output and/or could be used internally, e.g. for calibrating the PPG device 1.

For the skin tone measurement according to the first embodiment, it is possible to add a removable adapter so that the direct surface reflections between source and detector (light detected by the detector coming directly from the emitter) are avoided/minimized. This can be achieved by adding a special light blocker to guide the light towards the skin ( emitter and detector at same height). This could be needed when the measurements are performed in no contact with the skin, where diffused light in air will reach the detector without interacting with the skin surface first if emitter- detector are at the same height and a light guide/blocker is not used. This light guide could be a removable adapter with the shape of a hollow cylinder of a height at least half the distance between the skin and the emitter/detector surface where the emitters used for the measurement remain inside such structure.

In a second embodiment, the measurement to determine the skin tone according to the first embodiment could be varied by performing the measurement, when the skin/tissue is in contact with the tissue contact surface 2 (not at a certain distance from it). In this case, the skin tone could be derived from the DC portions of the two PPG signals of the two spectral bands. The same spectral bands as described above could be used or also different spectral bands.

Now, three different calibrations of the PPG device 1 are described. The PPG device 1 can work with one, two or all of these described calibrations or even without such a calibration. The calibration(s) are preferably done each time a tissue is brought in contact with the tissue contact surface 2. However, the calibration can also be stored, e.g. when the PPG device 1 is used always by the same user. Or the device can store different user profiles and the calibration is done once per user and then stored within the user profile.

A first calibration is a channel calibration (or path calibration). The anatomy and physiology of tissues 17 have a great impact on light-tissue interactions by influencing light scattering properties. The average depth of penetration depends on the wavelength, as well as the density and variations in the paths of the photons. The inventors found that the loss of signal observed in the simulations seems to be strongly related to the increase in skin thickness that occurs with a high Body Mass Index (BMI). The inventors suggest that this could indicate that the source-detector separation used in some devices may not be sufficient to sample quality vascular plexus at the same level for obese and non-obese individuals. Based on these observations, the inventors found that a longer source-detector separation in the PPG sensor allows for greater interrogation depth and helps to better capture blood volume changes in obese skin. However, since there are not only two types of skin, it is desirable to have multiple source-detector distances available in a PPG sensor 3 as proposed in the present invention. The inventors found out further on that also different skins can require different light intensities. E.g. a higher light intensity might be better suited for a longer path. Also for darker skin tones, where melanin content affects light absorption. To suit different scenarios, it is desirable to have a flexible system where the source-detector distance and/or the illumination intensity of the light emitters can be dynamically adjusted. While the state of the art shows already devices with two different source detector distances, this is not enough to cover the different types of skin. With the suggested design with a plurality of emitter units 6 (source) and a plurality of photodetectors 7 (detector), the multiple permutations of possible emitter-detector pairs allow a plurality of possible emitter-detector distances. Thus, this allows to select the optimal distance (and light intensity) for each type of tissue 17. The purpose of the channel calibration is to select the best optical paths for the subsequent PPG measurement of the tissue.

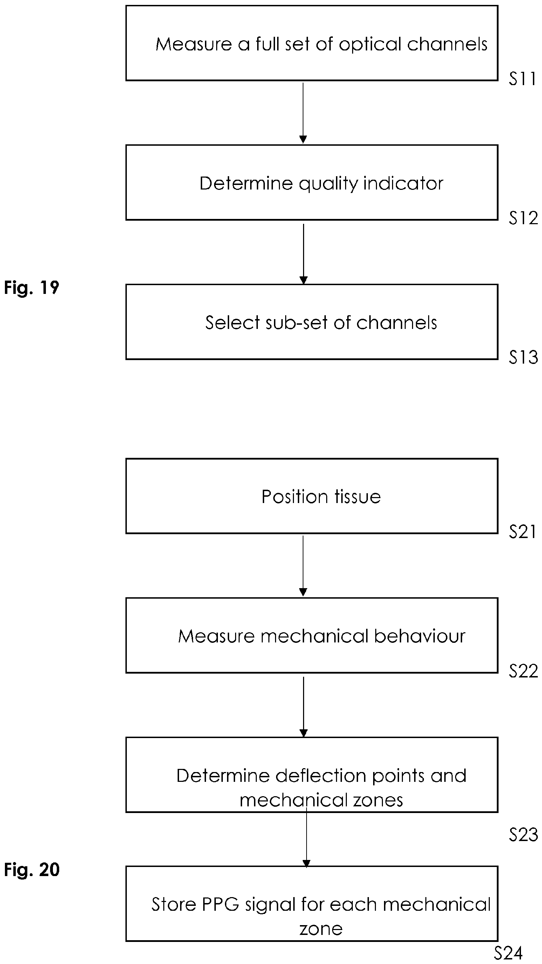

The channel calibration is illustrated in Fig. 19.

In step S11, the PPG sensor 3 performs a scan of a full set of optical channels. To scan or measure a set of optical paths means that the PPG signal for each optical path is measured. Thus, for each path, the light is emitted from the emitter (unit) 6/61, 62, 63, 64, 65 of the respective optical path, and detected by the photodetector 7 of the respective optical path. This light measurement of each path is done periodically (in a sample frequency) for a certain time period (of multiple sample periods) to measure a PPG signal of the respective optical path. The optical paths are measured/scanned preferably one after the other. Preferably, a full scan or measurement of the optical paths of the set of optical paths is done for each sample point. This scan/measurement is then repeated for a plurality of sample points so that a signal of a certain measurement period is obtained for the set of optical paths. Depending on the acquisition section/scheme/configuration, it would also be possible to detect more or all optical paths related to one emitter (unit) (and one light intensity) at the same time by detecting the PPG signals at a plurality of photodetectors 7, in parallel at the same time. However, the optical paths related to different emitter(s) should be scanned/measured consecutively, at least when the emitters 61, 62, 63, 64, 65 emit the same wavelength/spectral band and the detectors cannot distinguish these different spectral bands. The scan of the full set of Q optical paths will thus result in Q PPG signals. The full set of optical channels include preferably all possible emitter-detector pairs preferably with different light intensities and/or with different distances and/or with the different spectral bands/emitters of each emitter unit. The number of all optical channels/paths in the described embodiment above is thus the number M of emitter units 6 times the number N of photodetectors 7 times the number L of emitters 61, 62, 63, 64, 65 times the number P of different light intensities resulting in Q=MxNxLxP optical paths. However, the full set of optical channels can also mean a sub-set of all possible optical paths, for example only the optical channels which are potentially relevant for the subsequent PPG measurement of the tissue 17. For example, if the PPG device 1 is in a special measurement mode and only one processed parameter is determined in the processing means 5 and the one processed parameter requires maybe only the measurement with one emitter 61, 62, 63, 64, 65, the full set of optical paths would only comprise the Q optical path with Q=MxNxP. The step S1 1 results thus in one PPG signal for each optical path for the full set of optical paths.

In step S12, a quality indicator for each optical path of the full set is determined. The quality indicator for each optical path is determined based on the PPG signal of the respective optical path. The quality indicator of a PPG signal is preferably obtained based on the (raw) PPG signal x measured and based on a filtered PPG signal y. Possible quality indicators are the perfusion index, skewness, kurtosis, entropy, zero crossing rate and signal to noise ratio. The gold standard is the perfusion index calculated by

$$P_{SQI} = \left[ \frac{(y_{max} - y_{min})}{\|\overline{x}\|} \right] * 100$$

i.e. based on the ratio between the maximum amplitude between minimum and maximum value of the filter PPG signal and the mean value of the raw PPG signal x. In the present embodiment, the signal to noise ratio

$$N_{PQI} = \frac{\sigma^2_{signal}}{\sigma^2_{noise}}$$

where σ signal is the standard deviation of the absolute value of the filtered PPG signal and σ noise is the standard deviation of the raw PPG signal, actually showed to be particularly performant. However, other quality indicators can be obviously used. Thus, the quality indicator is calculated for each optical path based on its PPG signal. Preferably, all quality indicators (referring to the same quality indicator applied to the different PPG signals) of the full set of optical paths are stored. This allows to perform the scan only once and to make the selection in step S13 for each PPG measurement in S11 based on the stored quality indicators of all optical paths (if the same tissue is measured). However, the scanning and quality indicator determination in steps S11 and S12 can also be repeated for each PPG measurement.

In step S13, a sub-set of optical paths/channels are selected based on the calculated quality indicator. If the requirements for the processed parameter to be computed are not already considered in the full set of optical paths, the sub-set of optical paths is further based on these requirements of the PPG measurement for the processed parameter to be computed. For example, for a processed parameter which is measured only with a wavelength in one specific spectral band, the sub-set of optical paths is then chosen only among those specific spectral bands. The number of optical channels selected depends on the characteristics of the processed parameter to compute. For example, calculating pulse wave velocity requires at least two photodetectors 7 that share one or more common light emitter unit(s). Light emitter units 6 and photodetectors 7 must be aligned and separated at a certain distance between them, and this distance must be equidistant for the measurement to be effective. On the other hand, for SpO2 measurement, a photodetector 7 and two light emitters 61, 62, 63, 64, 65 of different wavelengths (typically red and NIR) (also in the same light emitter unit 6) are sufficient, while the distance between optical elements must guarantee a sufficiently large SNR or other quality indicator. Thus, the selection step S13 allows to select the optimal source-detector distance, light intensity and optical path location. Due to the distribution of the multiple photodetectors 7 and light emitter units 6 over the PPG sensor 3 and the possibility to measure the optical path between any pair of photodetectors 7 and light emitter units 6, the optical path can be dynamically selected for any type of tissue 17. This channel calibration is done preferably each time the tissue 17 is newly brought in contact with the tissue contact surface 2 and/or is moved in order to always use the optimal optical paths.

A second calibration is the skin tone calibration. Pigments in various types of human skin could affect the PPG signal quality and it could even invalidate physiological measurements. It was found out that there is a clear dependence between skin tone and percentage coverage of good quality beat-to-beat intervals (BBI), as well as improved BBI coverage and accuracy after physical activity, especially with people with darker skin tones.

The skin tone effect can induce a relative signal loss of more than 60% in some commercial devices. Therefore, it is preferred that the PPG device 1 determines the skin tone (see above) based on the PPG sensor 3 and calibrates the PPG device 1 based on the determined skin tone of the tissue 17 to be measured. The calibration of the skin tone can be realized in many ways. In one embodiment, the computed skin tone could be used to correct the processed parameters based on the skin tone. In another embodiment, the calibration could be that based on the skin tone, the optimal optical path for the subsequent PPG measurement is selected. As an example, for darker skin tones, optical paths with higher light intensity or shorter distances might be more appropriate. Also, shorter wavelengths work better than longer wavelengths on darker skin tones for the same optical path. If skin tone calibration is combined with an automatic optical path selection calibration, the parameters for automatically selecting the optical paths may vary based on the calculated skin tone.

A third calibration refers to tissue visco-elastic or mechanical calibration. The contact force between tissue contact surface 2 and tissue 17 greatly influences the PPG waveform. In order to increase the reliability in the interpretation of the physiological parameters extracted from PPG signals, effective methods that take into account the effects of contact force/pressure changes on the signal are required. In previous studies, global contact force compensation for a PPG signal using correction methods have been proposed, as well as methods based on maintaining constant pressure on the tissue to obtain the optimal PPG signal. However, this is not very practical for continuous measurements or for those that last very long. The wide inter-individual variability induces differences in blood vessel compliance, tissue visco-elastic characteristics and mechanical nonlinearities, making these methods impractical on a multiwavelength PPG signal. On top of that, local environmental conditions like ambient temperature and humidity, and different pathologies also influence the mechanical response of the tissue. For this reason, the calibration of the PPG device 1 based on the particular user's tissue mechanical response is proposed.

The calibration procedure is personalized and it mainly consists of varying the external force/pressure exerted on the measurement point before carrying out a new measurement session of the physiological parameters. Once the calibration has been carried out, it will not be necessary to repeat it during the time that a measurement session lasts. However, it would be advisable to carry out a periodic recalibration in ambulatory situations or during long-term measurements. The recalibration periods may be determined automatically by the PPG device 1 or may be performed at the discretion of the user or trained medical personnel. The criteria for the recalibration will be based on the determination of significant changes in the mechanical properties of the tissue with respect to the initial conditions after the initial calibration.

The initial mechanical conditions of the tissue can change, for example, as a consequence of changes in the initial measurement point, sudden, voluntary or accidental movements of the subject that induce displacements of the PPG 1 device with respect to the tissue. Also, under changes in temperature and humidity of the tissue and/or the environment.

This mechanical calibration/recalibration can be done manually by the user himself/herself (with the help of a trained operator), or automatically by means of a variable pressure means appropriately adapted for this task. Throughout the process, the signals involved will be continuously recorded to determine deflection/calibration points. The terms deflection point and calibration point are used as equivalents. These signals are all the sub-set of optical channels previously selected in S13 used for PPG. The capacitive sensor, or the signals provided by at least one auxiliary sensor used by a particular setup, or any method used to directly or indirectly estimate the exerted pressure can be used. Some physiological parameters and/or SQI derived from the AC or DC components of the PPG signal can be used as auxiliary variables to determine the calibration points. Various ways of performing the calibration can be used, but all must have in common the varying pressure exerted on the tissue 17.

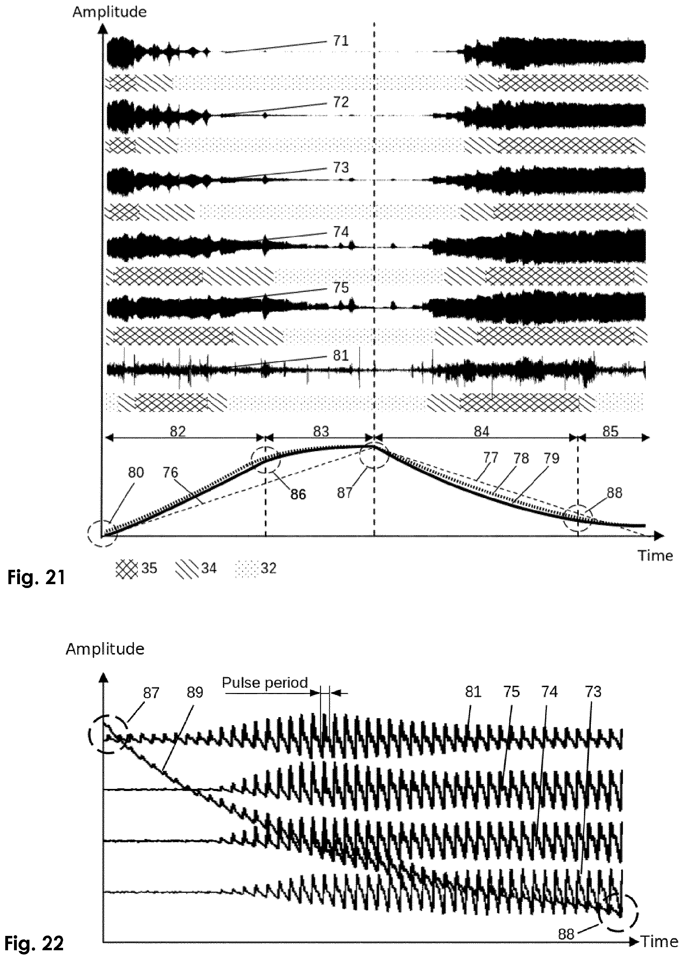

Figure 21 shows a several minutes representation of a multimodal graph were the AC components of PPG signals of five different wavelengths (71,..., 75), a tonometric signal 81, as well as the output response of a capacitive 4 and force sensor 8 can be seen. Below PPGs and tonometric signal, up to three different contact force zones (32, 34 and 35) can be observed. The increasing phase 76 and decreasing phase 77 of the applied force are also shown.

The contact force zones 32, 34 and 35 represent different quality zones of the exerted pressure 18. The quality zones indicate the PPG signal quality levels related with the applied force (of the varying pressure applied during the calibration). The zones 32 with maximal and minimal exerted forces represent a poor signal quality. The zone 34 represents acceptable signal quality and the pressure zone 35 represents an excellent PPG signal quality. The arrangement of the zones 32, 34 and 35 on the pressure scale varies, however, from tissue to tissue due to the different viscoelasticity properties of different tissue. This is why a visco-elastic or mechanical calibration improves the quality of the measurement.

Fig. 21 (bottom) also shows the average response curves (78, 79) of capacitive 4 and force 8 sensors over time when varying pressure is exerted on tissue 17. The exerted pressure or force is shown in Fig. 21 in curve 76, 77. So, fig. 21 allows to relate the mechanical response curve of the tissue 17 measured with the contact force sensors (4, 8) with the multiwavelength PPG 71, ..., 75 and the tonometric signal 81. The curve 78 (dotted) relates to the output of the capacitive sensor 4 and the curve 79 (continuous line) relates to the output of the contact force sensor 8. The capacitive sensor 4 or the contact force sensor 8 can be used equally for the tissue elastic calibration. In a preferred embodiment, both sensors 4, 8 are used for the calibration. The marked points 80, 86, 87 and 88 represent signal breakpoints or deflection points, which serve as reference thresholds for the calibration, and/or which help to identify relevant events related to the tissue visco-elastic properties in the multi-wavelength PPG signal. Starting at 80, the tissue contact with the tissue contact surface 2 is recorded, in section 82 the tissue behavior is mostly elastic (elastic zone). While in section 83, the tissue behavior is mainly visco-elastic (visco-elastic zone). In section 84 the tissue elastic retraction occurs (elastic retraction zone), while in 85 we are in the presence of the residual zone (residual zone), with a mostly visco-elastic behavior. Point 88 also serves to signal the end of the calibration. These response curves 78, 79 shown in Figure 21 can also be obtained from the DC components of the PPG signals without the use of additional sensors. However, it is better to use them for greater accuracy in the calibration.

As an example of the mechanical calibration, Fig. 22 shows the behavior of AC components of PPG signals 73, 74 and 75, and the tonometric signal 81 during the decreasing phase of the contact force 77 (between the inflection points 87 and 88). Curve 89 represents the capacitive 4/force sensor 8 signal output, from which the tonometric signal 81 is extracted and amplified. As it can be seen from the number of measurable PPG pulse periods displayed, the mechanical calibration can be completed in relatively short periods of time, from several seconds to a few minutes.

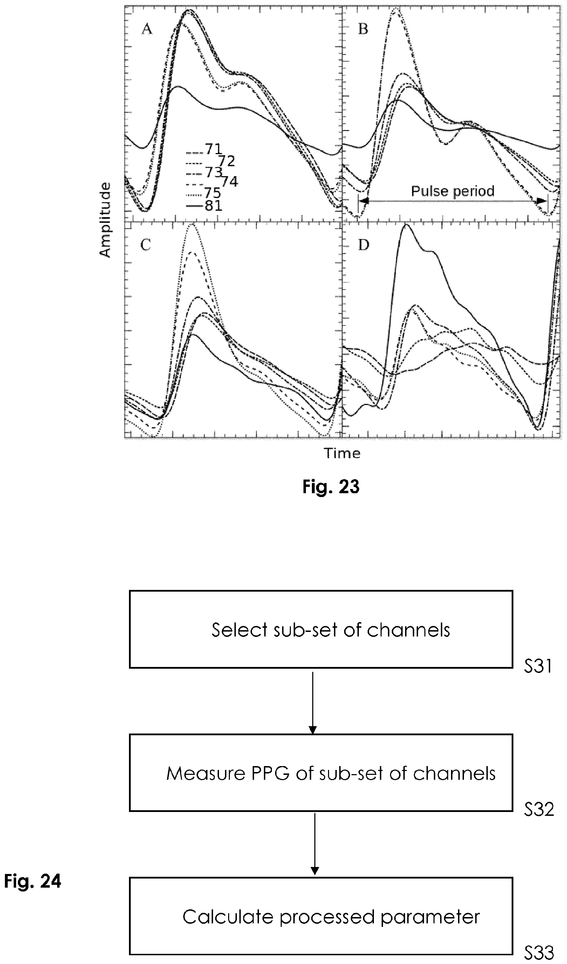

Figure 23 exemplifies (for a period of pulse or heartbeat) the possible changes in the contour of the AC components for the PPG signals of different wavelengths 71,..., 75 and for the tonometric signal 81. It can be observed that, for a low pressure (fig. 23A), for a medium-low pressure (fig. 23B), for a medium-high pressure (Fig. 23C) and for a high pressure (Fig. 23D) the contour of the signal changes because of the increasing applied pressure levels. It can be clearly seen that the worst contour for all wavelengths is obtained in Fig. 23D with the high pressure,while suitable contours for most applications can be obtained from low to medium-high pressure levels (Figures 23A, 23B and 23C). However, for the extraction of physiological parameters that depend to a greater extent on the quality of the pulse contour, the signal in Fig. 23B would be the most suitable.

Fig. 21, 22 and 23 show that actually the PPG signals 71, ..., 75 corresponding to different spectral bands react differently on the exerted or applied pressure 18. Fig. 21 shows the curve 76 (dashed) of the variation of the exerted pressure 18 over time during the increasing phase, and curve 77 (dashed) during the decreasing phase, showing also how the AC components of PPG signals 71, ..., 75 of five different spectral bands and the tonometric signal 81 react to these force phases/levels. The wavelength of curve 71 is the shortest and the wavelength of curve 75 is the longest. Thus, the curves 71 to 75 increase in wavelength. The curves 71, ..., 75 correspond to the optical paths of the emitters 61, ..., 65, respectively. As it can be seen, the PPG signals of the longer wavelengths are less sensitive to the increase in pressure than the shorter wavelengths. The pressure zones 35 show the highest amplitude and highest quality AC pulses of the PPG signals 71, ..., 75, and tonometric signal 81. Differences in amplitude and SQI can also be observed for the same value of contact force between the signals obtained during the increasing and decreasing phases of the contact force, which suggests that the viscoelastic behavior of the tissue induces hysteresis in the response of the PPG signals 71, ..., 75, and tonometric signal 81.

Fig. 20 shows the process/method of the tissue elastic calibration.

In step S21, tissue 17 is positioned on the tissue contact surface 2. The tissue contact on the tissue contact surface 2 can be detected by device 1 (as described above). Once the contact is detected and/or the start signal for the calibration is received, the subsequent steps can be started.

In step S22, the mechanical behaviour is measured while the pressure of the tissue 17 on the tissue contact surface 2 is varied.

The variation of the pressure of the tissue 17 on the tissue contact surface 2 refers to the pressure with which the tissue 17 is pressed against the tissue contact surface 2. This will also be shortly referred as the applied pressure. This applied pressure is preferably increased and subsequently decreased as shown in the pressure profile 76 and 77, preferably in a triangle function. However, other pressure profiles for the pressure variation are possible. This applied pressure must be distinguished from the measured pressure at the sensor(s) 4 and/or 8 as it can be seen in Fig.21, which shows in the bottom graph the measured force curve 78 and 79 with sensors 4 and 8, respectively, over time, while the curves 76 and 77 shows the contemporaneous change of the applied pressure over the same time. The change of the pressure should be slow enough that at least one, preferably at least two or three heart cycles can be measured for the substantially the same applied pressure.

The measurement of the mechanical behaviour comprises preferably at least one of the following measurements: the capacitive value measured by the capacitive sensor 4, the contact force measured by the contact force sensor 8 and the PPG signal (of the different wavelengths or spectral bands) obtained by the PPG sensor 3. The measurement of the mechanical behaviour comprises preferably the PPG signal (of the different wavelengths or spectral bands) obtained by the PPG sensor 3 and at least one of the following measurements: the capacitive value measured by the capacitive sensor 4 and the contact force measured by the contact force sensor 8.

In step S23, deflection/calibration points are determined based on the measured mechanical behaviour. A deflection point is a measured pressure point at which the mechanical behaviour of the tissue changes, e.g. from elastic behaviour to visco-elastic behaviour or vice versa. Preferably, the deflection points are determined based on the measurement of the capacitive sensor 4 and/or the contact force sensor 8. However, it would also be possible to detect the deflection points based on the AC and/or DC components of PPG signal. The deflection points 80, 86, 87, 88 can be determined from the curve of the sensor 4 and/or 8 over the time of the pressure variation. The deflection points 80, 86, 87, 88 can be determined from the curve of the capacitive sensor 4 and/or the contact force sensor 8, in particular from its shape. When the applied force 76, 77 are actively controlled, the points 80, 87, 88 can also be determined based on the applied pressure control. The time point of the deflection points 80, 86, 87, 88 can be used to determine the different mechanical zones in the next step. The deflection points 80, 86, 87, 88 can be further used to measure the pressure value and/or capacitance value 78, 79 at the deflection points 80, 86, 87, 88.

In step S24, different mechanical zones are defined based on the deflection points 80, 86, 87, 88, and the PPG signal(s) (for the different wavelengths) of each mechanical zone and/or a feature of the PPG signal(s) are/is stored. Preferably, a typical waveform of the PPG signal for each mechanical zone is determined. Preferably, the quality (index) indicator(s) for the PPG signal of each mechanical zone (and each wavelength) is determined and stored. The different mechanical zones comprise at least one optimal mechanical zone which refers to the mechanical zone in which the PPG signal should be measured, i.e. the waveform of the PPG signal is optimal in this mechanical zone. The different mechanical zones comprise at least one further mechanical zone with less optimal measurement characteristics for acquiring the PPG signal, i.e. the waveform of the PPG signal is less optimal. The further mechanical zones could be further distinguished in acceptable zone in which the quality/waveform of the PPG signal is maybe not optimal but still acceptable and not acceptable zones in which a reasonable waveform of the PPG signal cannot be determined.

Preferably, at least one correction function is computed based on the PPG signals, preferably based on their typical waveforms, of the different mechanical zones. A correction function from a waveform of the optimal mechanical zone to a waveform of a first mechanical zone defines how the waveform of the first mechanical zone must be changed to obtain the waveform of the optimal mechanical zone (abbreviated: correction function of the first mechanical zone). The at least one correction function comprises a plurality of correction functions, preferably one correction function for each further mechanical zone or for each acceptable mechanical zone.

This mechanical calibration of the device allows to measure the PPG signal for any applied pressure or at least for a larger applied pressure range. Even if the applied pressure is not in the optimal pressure zone 35, the calibration allows to correct the PPG signal. Thus, the calibration combines the advantages of the two methods of the state of the art.

During the measurement of the PPG signal, this mechanical calibration is used in the following way.

The mechanical zone of the tissue 17 is determined based on the PPG signal measured/stored. This can be done by comparing the measured PPG signal or a parameter of the PPG signal with the (parameter of the) PPG signal of the different mechanical zones to detect the mechanical zone of the tissue 17 based on the comparison. The parameter of the PPG signal can be for example the quality (index) indicator of the PPG signal. By comparing the quality (index) indicator of the measured PPG signal with the quality (index) indicator of the mechanical zones, the mechanical zone can be determined. The quality (index) indicator can be stored for each mechanical zone in the calibration or can be calculated from the stored PPG signals of the mechanical zones. Alternatively or additionally, the waveform of the measured PPG signal (for each wavelength) can be compared with the waveform of the PPG signal of the mechanical zones (and the compared wavelength) to determine the mechanical zone while measuring the PPG signal. In one embodiment, the quality (index) indicator of the measured PPG signals of all wavelengths with the stored quality (index) indicators of all wavelengths provides a good estimator for mechanical zone. Alternatively or in addition, different features, parameter or quality (index) indicators can be used to further improve the detection robustness. The mechanical zone whose PPG signal waveform is most similar to the waveform of the measured PPG signal or whose stored features/parameters/quality (index) indicators are most similar to the ones measured can be chosen as the mechanical zone of the measured PPG signal.

If the mechanical zone determined is the optimal mechanical zone, the PPG signal measured can be used without a correction of the PPG signal.

If the mechanical zone determined is one of the further mechanical zone, the PPG signal measured is corrected based on the mechanical calibration, preferably based on the correction function of the detected mechanical zone. The correction function of the detected mechanical zone is applied on the PPG signal measured . Alternatively, or additionally, the applied pressure could be changed so that the PPG signal measured returns in the optimal mechanical zone. If the applied pressure is actively controlled, the applied pressure is simply changed. Otherwise, a user output could indicate how the applied pressure is to be changed, i.e. increased or decreased. This can be an audio or visual or haptic output indicating to change the applied pressure.

The term waveform of the PPG signal is used in here for the shape of the PPG signal of one optical channel corresponding to the duration of one heartbeat.

It has been shown that the prior calibration and the detection of the mechanical zone of the tissue 17 of the respective PPG measurement from the waveform of the measured PPG signal is much more reliable than from a direct measurement of the contact pressure. On the other side, this method does not require for each measurement to go through the complete pressure range to find the optimal mechanical zone, just once for the mechanical calibration. This does obviously not exclude to redo the mechanical calibration from time to time.

Subsequently, the operation of the PPG device 1 for determining a certain processed parameter is described with Fig. 24. As exemplary processed parameter the determination of the pulse wave velocity will be described.

In step S31, the processing means 5 selects a sub-set of optical paths (or channels) for the PPG measurement of the at least one processed parameter. At least one processed parameter to determine requires the measurement of the PPG signal with certain condition, e.g. for the wavelength(s), for the obtained PPG waveform, for the number of PPG signals required, for the location distribution of the different optical paths, etc. The processed parameter might be calculated based on one PPG signal of a certain wavelength. This one PPG signal could be calculated based on one single optical channel with the certain wavelength or based on an average signal of a group of PPG signals with the certain wavelength (and having maybe other parameters like distance light intensity in common). Such an average signal might be obtained based on post-processing (digitally averaging the individually obtained signals) or by analogue averaging (simply measuring the optical paths at the same time). Indeed, the processed parameter can be computed based on any linear combination of optical paths. E.g. the pulse wave velocity requires two PPG signals which are obtained from the same light emission with two distinct photodetectors 7. The same light emission is preferably obtained by one common light emitter (unit 6) 61, 62, 63, 64, 65. However, the same light emission could also be obtained by a group of more than one emitter unit 6 at least two photodetectors that share one or more common light emitters. Also, each of the two photodetectors 7 might be one photodetector 7 or a group of multiple photodetectors 7. The photodetectors 7 are preferably aligned, i.e. are chosen in one line between the two photodetectors 7 and the light emitting unit 6, e.g. are chosen all in the same row or column. The Photodetectors 7 are chosen as far as possible to get a larger distance for calculating the speed. The sub-set of channels is preferably not only chosen based on the requirements of the processed parameter(s) to compute, but also on the signal quality as determined in the channel calibration explained above. If the at least one processed parameter to be computed comprises two or more processed parameters, the different optical paths for calculating all processed parameters can be selected together.

In this case, optical paths which are needed for two or more processed parameters must be measured only once.

In step S32, the PPG signals of the selected sub-set of optical paths or channels is measured. Preferably, the PPG signals of these channels are continuously measured. Depending on the processed parameter to be computed, the PPG signals can be measured with different sampling rates. The heart rate might require a lower sampling rate than another processed parameter which is determined from the shape of the PPG signal waveform.

In step S33, (each of) the at least one processed parameter is calculated based on the at least one PPG signal measured in step S32. As common in this field, the measured PPG signals are preferably filtered to remove noise before they are used for further processing. However, for some processed parameters, it might be also that the raw PPG signal is used. The at least one processed parameter is preferably output by the output means 27.

The processing means 5 is also configured to determine other processed parameters which are based not on the PPG sensor 3, but on other sensors.

The processing means 5 might be configured to determine the tonometric signal 81 based on the contact force sensor 8 and/or based on the capacitive sensor 4. Tonometry is a non-invasive technique for continuous measurement of pressure. This signal could be used as auxiliary solutions for continuous non-invasive measurement of blood pressure. A pulsating pressure variation contained in a blood vessel 20 exerts an outward force 19 that leads to a local displacement of the tissue surface 21. When the PPG device 1 is gently pressed with application force 18 onto the tissue 17 deforming it slightly, the displacement due to the exerted force 19 changes proportionally the electric field of the capacitive sensor 4 and/or the contact pressure measured by the contact force sensor 8 (see Figure 16). Thus, the tonometric signal 81 can be measured by the variations of the capacitance of the capacitive sensor 4 and/or by the changes of the contact force measured by the contact force sensor 8. Thus, the acquired signal contains the relative change of the pressure during the heart cycle. The amplitude and waveform of the tonometric signal 81 depends on the pressure 18 exerted by the multiparametric PPG device 1 on the tissue 17, the configuration and sensitivity of the capacitive sensor 4 and/or the contact force sensor 8, as well as the selected measurement point (see Figure 22). For that reason, the calibration to absolute blood pressure values must be performed. The calibration of the pressure applied could be done automatically by a variable pressure means. Such a variable pressure means could be a belt whose length can be varied by a motor. When the belt encloses a body part holding the PPG device 1 with the tissue contact surface 2 against the tissue 17, the varying length of the belt could vary the pressure 18 applied. However, the changing application pressure 18 could also be achieved by user instructions, providing the user with instructions to correct the pressure with which he presses against the tissue contact surface 2.

The processing means 5 might be configured to determine the respiration signal based on the capacitive sensor 4. Respiration monitoring allows early detection of diseases and disorders that can manifest suddenly in a life-threatening episode. The respiration signal can be determined from the PPG signal for example by a principal component analysis and/or by a band pass filter chosen in the usual frequency of the respiration. The obtained respiration signal could be verified with the respiration components detected in the PPG signal to increase the precision of the respiration signal. The obtained respiration signal could also be used to filter out the respiration components from the PPG signal(s). Capacitive sensing is an inexpensive method for long-term and short-term respiration detection.

The (other) processed parameters determined by the processing means 5 can be output by the output means 27. The output means 27 can be simply an interface to give out the determined signal/value. This can be a wired or wireless interface. Examples for a wired interface can be a USB socket. Examples for a wired interface can be a cellphone network interface, like a GSM module, a Bluetooth interface, a WIFI interface, etc. The output means 27 can alternatively or additionally comprise a display for showing the determined signal(s) or value(s). The output means 27 could also be a storage for simply storing the processed parameters.

Due to the multiple possibilities to use the described PPG device 1, it can also be called a multiparametric PPG device.

It should be understood that the present invention is not limited to the described embodiments and that variations can be applied without going outside of the scope of the claims.

## Claims

1. Device for measuring a photoplethysmogram (PPG) of a tissue, the device comprising:

   a tissue contact surface configured to be contacted by the tissue;
   a plurality of light emitter units for emitting light in the tissue contact surface, wherein the plurality of light emitter units comprise at least three light emitter units;
   a plurality of photodetectors for detecting in the tissue contact surface the light emitted by the light emitter units, wherein the plurality of photodetectors comprise at least three photodetectors;
   a signal unit for measuring PPG signals of different optical paths provided by all possible pairs of light emitter units and photodetectors.

2. Device according to the previous claim, wherein each of the light emitter units is configured to emit the light of the same spectral band.

3. Device according to one of the previous claims, wherein the plurality of light emitter units and the plurality of photodetectors are arranged in a grid of rows and columns.

4. Device according to the previous claim, wherein a row and/or a column of the grid comprises alternately a light emitter unit and a photodetector.

5. Device according to the previous claim, wherein a neighboring row and/or column of the row and/or column show the same pattern off-set by one position.

6. Device according to one of the previous claims, wherein each of the plurality of light emitter units comprise a spectral number of sub-light emitter units emitting light of a different spectral band, wherein the plurality of different optical paths comprises the spectral number of optical paths corresponding to the different sub-light emitter units between each emitter-detector couple of the plurality of light emitter units and the plurality of photodetectors.

7. Device according to one of the previous claims, wherein the plurality of light emitter units are configured to emit light at a first light intensity and at a second light intensity different from the first light intensity, wherein the plurality of different optical paths comprises for each emitter-detector pair at least one first intensity optical path using emitted light at the first light intensity and at least one second intensity optical path using emitted light at the second intensity.

8. Device according to one of the previous claims, wherein the processing means is configured to perform a channel calibration comprising to:

    measure a PPG signal of all optical paths of a full set of optical paths,
    calculate a quality indicator for all optical paths based on their PPG signals, and
    select a sub-set of optical paths based on the quality indicators of the optical paths,
    wherein the processing means is configured to perform a signal processing comprising to:

        measure the PPG signal(s) of the sub-set of optical paths, and
        determine a processed parameter based on the measured PPG signal(s).

9. Device according to the previous claim comprising an auxiliary sensor configured to measure at least one measurement parameter influencing the PPG signal; wherein the signal processing comprises to determine periodically the processed parameter based on the PPG signal measured, wherein the processing means is configured to re-perform the channel calibration, when the measured auxiliary parameter indicates a change necessitating a new channel calibration, wherein the signal processing comprises, after having re-performed the channel calibration, to measure PPG signal(s) of a new sub-set of optical paths determined in the re-performed channel calibration and periodically determining the processed parameter based on the PPG signal(s) of the new sub-set of optical paths.

10. Device according to claim 8 or 9, wherein the signal processing comprises to determine periodically the processed parameter based on the PPG signal measured, wherein the processing means is configured to re-perform the channel calibration, when a quality indicator of the PPG signal(s) of the sub-set of optical paths indicates a deterioration of the quality of the PPG signal(s).

11. Device according to one of the previous claims comprising a capacitive sensor in the tissue contact surface to measure a capacitive value of the tissue in contact or in vicinity of the tissue contact surface, wherein the capacitive sensor comprises at least one capacitive electrode arranged in the tissue contact surface, wherein the capacitive sensor is configured for each capacitive electrode to apply a drive signal generated by a drive circuit on the capacitive electrode and measure the capacitive value of the capacitive electrode on the same capacitive electrode by an acquisition circuit such that the drive circuit and the acquisition circuit are galvanically connected by the capacitive electrode.

12. Device according to one of the previous claims, a skin tone of the tissue is measured based on the PPG signal obtained by the PPG sensor.

13. Device according to the previous claim, wherein the skin tone is measured with the following steps:

    positioning the tissue at a distance to the tissue contact surface,
    emitting from the light emitting unit light of a first spectral band and measuring the emitted light reflected from the tissue at the photodetector as first reflected tone light,
    emitting from the light emitting unit or another light emitting unit of the plurality of light emitting units light of a second spectral band and measuring the emitted light reflected from the tissue at the photodetector or at another photodetector of the plurality of photodetectors as second reflected tone light,
computing the skin tone based on the first and second reflected tone light.

14. Device according to the previous claim, wherein the measured skin tone is used to calibrate the device for the PPG measurement of the tissue.

15. Device according to one of the previous claims, wherein the processing means is configured to perform a signal processing to compute a processed parameter, wherein the processing means is configured to perform a mechanical tissue calibration, wherein the mechanical tissue calibration comprises to measure at least one PPG calibration signal with the PPG sensor and optionally an auxiliary calibration signal with an auxiliary sensor while the pressure of the tissue applied on the tissue contact surface is changed, wherein the mechanical tissue calibration comprises to detect different mechanical zones of the tissue based on the PPG calibration signal or based on the auxiliary calibration signal, wherein the mechanical tissue calibration comprises further storing at least one waveform of the PPG calibration signal for each detected mechanical zone, wherein the signal processing comprises the measurement of a PPG measurement signal by the PPG sensor and a detection of the mechanical zone of the tissue during PPG measurement signal based on the waveform stored for the detected mechanical zone.

**Fig. 1**

**Fig. 2A**

**Fig. 2B**

**Fig. 3**

**Fig. 4**

**Fig. 5A**

**Fig. 5B**

**Fig. 6**

**Fig. 7**

**Fig. 8**

**Fig. 9**

Fig. 10

Fig. 11

Fig. 12

**Fig. 13A**

**Fig. 13B**

**Fig. 13C**

**Fig. 14**

**Fig. 15**

**Fig. 16**

Amplitude

22

23

26

21

25

Time

**Fig. 17**

24

| Position tissue |
| --- |

S1

| Measure with first spectral band |
| --- |

S2

| Measure with second spectral band |
| --- |

S3

| Compute skin tone |
| --- |

S4

**Fig. 18**

**Fig. 19**

Measure a full set of optical channels

S11

Determine quality indicator

S12

Select sub-set of channels

S13

Position tissue

S21

Measure mechanical behaviour

S22

Determine deflection points and mechanical zones

S23

**Fig. 20**

Store PPG signal for each mechanical zone

S24

**Fig. 21**

**Fig. 22**

Fig. 23

Fig. 24

Select sub-set of channels
S31

Measure PPG of sub-set of channels
S32

Calculate processed parameter
S33

Europäisches
Patentamt

European
Patent Office

Office européen
des brevets

## EUROPEAN SEARCH REPORT

Application Number

EP 23 15 5692

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/273207 A1 (MOHAMMADI SAEED [US] ET AL) 1 September 2022 (2022-09-01) | 1-3,6-8 | INV. A61B5/0295 |
| Y | * paragraphs [0020] - [0024], [0044], [0045], [0137] - [0138]; figure 1D * | 4,5, 11-14 | A61B5/026 A61B5/024 |
| A | | 9,10,15 | A61B5/1455 A61B5/00 |
| | ----- | | |
| X | US 2017/311825 A1 (WEEKLY KEVIN PU [US] ET AL) 2 November 2017 (2017-11-02) | 1-3,6-8 | |
| Y | * paragraphs [0043], [0044], [0072], [0073], [0081], [0104] - [0105], | 4,5, 11-14 | |
| A | [0111]; figures 1E, 1F, 4B * | 9,10,15 | |
| | ----- | | |
| Y,D | EP 3 342 336 A1 (SAMSUNG ELECTRONICS CO LTD [KR]) 4 July 2018 (2018-07-04) * paragraphs [0034] - [0039] * | 11 | |
| | ----- | | |
| Y | US 10 687 717 B1 (PETERSON RUI L [US] ET AL) 23 June 2020 (2020-06-23) * figure 8B * | 4,5 | |
| | ----- | | |
| Y | CN 106 073 737 B (FITBIT INC) 23 November 2018 (2018-11-23) * paragraph [Ambientlightandskintone] * | 12-14 | TECHNICAL FIELDS SEARCHED (IPC) A61B |
| | ----- | | |

The present search report has been drawn up for all claims

| Place of search | Date of completion of the search | Examiner |
|---|---|---|
| Munich | 18 April 2023 | Gentil, Tamara |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 23 15 5692

This annex lists the patent family members relating to the patent documents cited in the above-mentioned European search report.
The members are as contained in the European Patent Office EDP file on
The European Patent Office is in no way liable for these particulars which are merely given for the purpose of information.

18-04-2023

| Patent document cited in search report | | | Publication date | Patent family member(s) | | Publication date |
|---|---|---|---|---|---|---|
| US 2022273207 | A1 | | 01-09-2022 | US | 11331016 B1 | 17-05-2022 |
| | | | | US | 2022273207 A1 | 01-09-2022 |
| US 2017311825 | A1 | | 02-11-2017 | CN | 109195510 A | 11-01-2019 |
| | | | | EP | 3448249 A1 | 06-03-2019 |
| | | | | US | 2017311825 A1 | 02-11-2017 |
| | | | | US | 2018177416 A1 | 28-06-2018 |
| | | | | US | 2020138309 A1 | 07-05-2020 |
| | | | | US | 2021137397 A1 | 13-05-2021 |
| | | | | WO | 2017190051 A1 | 02-11-2017 |
| EP 3342336 | A1 | | 04-07-2018 | CN | 108236460 A | 03-07-2018 |
| | | | | EP | 3342336 A1 | 04-07-2018 |
| | | | | EP | 3943001 A1 | 26-01-2022 |
| | | | | JP | 7125255 B2 | 24-08-2022 |
| | | | | JP | 2018102906 A | 05-07-2018 |
| | | | | KR | 20180076050 A | 05-07-2018 |
| | | | | US | 2018177413 A1 | 28-06-2018 |
| US 10687717 | B1 | | 23-06-2020 | US | 10687717 B1 | 23-06-2020 |
| | | | | US | 2020315473 A1 | 08-10-2020 |
| CN 106073737 | B | | 23-11-2018 | CN | 104207756 A | 17-12-2014 |
| | | | | CN | 106073737 A | 09-11-2016 |
| | | | | CN | 106333667 A | 18-01-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3342336 A1

- WO 2018231672 A1